(19) **Europäisches Patentamt / European Patent Office / Office européen des brevets**

(11) **EP 2 842 476 B1**

(12) **EUROPEAN PATENT SPECIFICATION**

(45) Date of publication and mention of the grant of the patent:
**05.04.2017 Bulletin 2017/14**

(21) Application number: **13781796.1**

(22) Date of filing: **26.04.2013**

(51) Int Cl.:
*A61B 1/00* (2006.01)  *A61B 5/06* (2006.01)
*A61B 5/07* (2006.01)  *A61B 1/04* (2006.01)

(86) International application number:
**PCT/JP2013/062505**

(87) International publication number:
**WO 2013/162033 (31.10.2013 Gazette 2013/44)**

(54) **POSITION-DETECTING DEVICE, CAPSULE ENDOSCOPE SYSTEM, AND POSITION-DETECTING PROGRAM**

POSITIONSBESTIMMUNGSVORRICHTUNG, KAPSELENDOSKOPSYSTEM UND POSITIONSBESTIMMUNGSPROGRAMM

DISPOSITIF DE DÉTECTION DE POSITION, SYSTÈME D'ENDOSCOPE À CAPSULE ET PROGRAMME DE DÉTECTION DE POSITION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.04.2012 JP 2012101447**

(43) Date of publication of application:
**04.03.2015 Bulletin 2015/10**

(73) Proprietor: **Olympus Corporation**
**Tokyo 192-8507 (JP)**

(72) Inventors:
• **HIGAKI, Naoya**
**Tokyo 151-0072 (JP)**

• **HOMAN, Masatoshi**
**Tokyo 151-0072 (JP)**
• **HASEGAWA, Jun**
**Tokyo 151-0072 (JP)**

(74) Representative: **Gunzelmann, Rainer**
**Wuesthoff & Wuesthoff**
**Patentanwälte PartG mbB**
**Schweigerstraße 2**
**81541 München (DE)**

(56) References cited:
WO-A1-2012/042987    JP-A- H08 500 441
JP-A- 2006 212 051    JP-A- 2006 212 051
JP-A- 2007 283 001    US-A1- 2002 193 685
US-A1- 2006 183 993    US-A1- 2008 312 501

**Description**

Field

The present invention relates to a position detection device that detects a position of a capsule endoscope in a subject, a capsule endoscope system including the position detection device, and a position detecting program.

Background

In the endoscopic field, a capsule endoscope containing an imaging function, a wireless communication function, and the like in a capsule-shaped casing formed in size insertable into the digestive tract of a subject such as a patient is conventionally known. The capsule endoscope is swallowed from the mouth of the subject and then moves in the subject such as in the digestive tract by the peristaltic movement and the like. Images of the inside of the subject are sequentially captured to generate image data. The image data are sequentially transmitted wirelessly.

The image data transmitted wirelessly from the capsule endoscope are received by a receiving device provided to the outside of the subject, and stored in a memory embedded in the receiving device. After the end of the examination, the image data accumulated in the memory of the receiving device are captured in an image display device. An observer such as a doctor observes an organ image and the like that are displayed by the image display device, and diagnoses the subject.

The capsule endoscope moves in the body cavity by the peristaltic movement and the like and accordingly it is required to correctly identify where in the body cavity the image data transmitted wirelessly from the capsule endoscope are captured. Hence, known is a medical system and the like that receive electromagnetic waves transmitted by the capsule endoscope by a plurality of receiving antennas provided outside the body cavity, and estimate the position of the capsule endoscope with the received strength of a plurality of received wireless signals, and the like (for example, see Patent Literatures 1 to 3).

For example, Patent Literature 1 discloses a technology for estimating the position of a capsule endoscope based on the received strength of an antenna, estimating travel speed and travel direction of the capsule endoscope in predetermined situations such as where reception is poor, and estimating the position of the capsule endoscope based on these travel speed, travel direction, and received strength.

Moreover, Patent Literature 2 discloses a magnetic guidance medical system that controls the position and posture of a capsule-shaped medical device in the body by magnetism. In the magnetic guidance medical system, third-angle projection is applied to calculate a three-dimensional position of the capsule-shaped medical device based on the received strength of a wireless signal transmitted from the capsule-shaped medical device.

Moreover, Patent Literature 3 discloses a capsule-shaped medical device that estimates the position of a capsule endoscope in the body and calculates the locus. In the capsule-shaped medical device, the estimation of the position and orientation of an antenna contained in a capsule endoscope is repeated by the Gauss-Newton method.

US 2006/183993 A1 discloses a system for locating an in-vitro sensor including a location detecting unit to locate the in-vivo image sensor over time; and a data modifying unit to modify data sampled by the location detecting unit based on information sensed by the in-vivo image sensor. The system estimates the location of the sensor signal by detecting the strength of the signal from several antennas. Other types of data sensed from the signal source may be used to confirm or modify the generated location data, such as image data, temperature, pH, acceleration, and oxygen saturation.

US 2002/193685 A1 discloses a system for locating and tracking the position of a target, such as a tumor within a body, by one or more excitable markers. An external source excites the markers, which in turn emit a signal to an array of sensors spaced apart in a known geometry. A computer is coupled to the sensors and configured to use the marker measurements to identify a target isocenter within the target. The computer compares the position of the target isocenter with the location of the machine isocenter. The distance from each sensor to a marker is calculated using signal strength and iterated for a best fit solution, providing a sphere of possible locations is determined around each sensor. The intersection of four such overlapping spheres defines a single point where the marker is located.

US 2008/312501 A1 teaches a system for locating an endoscopic capsule which transmits a high-frequency signal from within a living body to a plurality of antennas. A CPU performs an estimation process on the initial location and orientation so as to compute new estimated location and orientation and update the initial location and orientation to the new location and orientation. The CPU similarly performs the estimation process on the updated location and orientation. The CPU performs accurate location estimation by repeatedly performing the estimation process until the amount of shift of the location computed and updated by the estimation process reaches a sufficiently small value.

Citation List

Patent Literature

Patent Literature 1: Japanese Laid-open PatentPublication No. 2008-99734
Patent Literature 2: Japanese Laid-open Patent Publication No. 2006-68501
Patent Literature 3: Japanese Laid-open Patent Publication No. 2007-283001

Summary

Technical Problem

However, a transmitting antenna that is built-in to a capsule endoscope and transmits a wireless signal generally has directivity, and therefore it is necessary to consider an orientation of the transmitting antenna, i.e., an orientation of the capsule endoscope, for performing position estimation with high accuracy.

For that point, Patent Literature 3 discloses that orientation information can be used for locus calculation process in addition to the position information of the transmitting antenna of the capsule endoscope. However, Patent Literature is silent on how the orientation information is used.

Moreover, in order to perform calculation with high accuracy, an amount of calculation generally increases; therefore, it becomes difficult to perform the calculation of the estimation at a high speed.

The present invention has been made considering the above and an object thereof is to provide a position detection device, a capsule endoscope system and a position detecting program that can detect the position of the capsule endoscope with higher accuracy than before while cutting the calculation amount.

Solution to Problem

To solve the above-described problem and achieve the object, a position detection device according to the present invention detects a position of a capsule endoscope in a subject, the capsule endoscope being introduced into the subject and moving in the subject, based on received strength of signals transmitted from the capsule endoscope at a plurality of receiving antennas. The position detection device includes: a region acquiring unit that obtains distances between the receiving antennas and the capsule endoscope based on the received strength of the signals received by the plurality of receiving antennas, and acquires a region where at least two spheres of a plurality of spheres with the receiving antennas as centers and the distances corresponding respectively to the receiving antennas as radii overlap with one another; an orientation estimating unit that estimates orientations of the capsule endoscope at a plurality of points in the region based on positional relationships between the points and the receiving antennas, and the received strength of the signals received by the receiving antennas; and a position determination unit that determines the position of the capsule endoscope in the subject based on the positions of the points and the orientations of the capsule endoscope.

In the above-described position detection device, the orientation estimating unit acquires a candidate region of a point at which a vector representing the orientation of the capsule endoscope passes based on a direction of the receiving antenna, a direction from the point to the receiving antenna, and the received strength at the receiving antenna, and determines, as the point at which the vector passes, a position where the candidate regions of the point at which the vector passes, the candidate regions being acquired respectively for the plurality of receiving antennas, overlap with one another.

In the above-described position detection device, the position determination unit calculates theoretical values of the received strength of the signals received by the plurality of receiving antennas based on the positions of the points and the orientations of the capsule endoscope in a case of assuming that the capsule endoscope exists at the points, as well as acquiring measured values of the received strength at the plurality of receiving antennas, and detects a point having a minimum error between the theoretical value and the measured value as the position of the capsule endoscope out of the plurality of points.

In the above-described position detection device, the error is a sum of absolute values of differences or a sum of squared residuals between the theoretical values and the measured values.

The above-described position detection device further includes a locus calculator that calculates a moving locus of the capsule endoscope based on the position of the capsule endoscope determined by the position determination unit.

The above-described position detection device further includes an image display unit that displays the moving locus of the capsule endoscope in the subject, the moving locus being calculated by the locus calculator.

A capsule endoscope system according to the present invention includes a capsule endoscope that is introduced into a subject and moves in the subject to acquire image information inside the subject, and the above-described position detection device.

In the above-described capsule endoscope system, the receiving antennas are antennas that use only main polarization.

In the above-described capsule endoscope system, the receiving antennas are dipole antennas.

A capsule endoscope system according to the present invention includes: a capsule endoscope that is introduced into a subject and moves in the subject to acquire image information inside the subject; a receiving device including a plurality of antennas for receiving a signal including the image information transmitted from the capsule endoscope, a region acquiring unit for obtaining distances between the receiving antennas and the capsule endoscope based on received strength of the signals received by the plurality of receiving antennas, and acquiring a region where at least two spheres of a plurality of spheres with the receiving antennas as centers and the distances corresponding respectively to the receiving antennas as radii overlap with one another, an orientation estimating unit for estimating orientations of the capsule endoscope at a plurality of points in the region based on positional relationships between the points and the receiving antennas, and the received strength of the signals received by the receiving antennas, and a position determination unit for determining a position of the capsule endoscope in the subject based on the positions of the points and the orientations of the endoscope; and an image display unit that acquires the image information and position information indicating the position of the capsule endoscope corresponding to the image information from the receiving device and displays an image corresponding to the image information and the position of the capsule endoscope.

A position detecting program according to the present invention is executed by a position detection device for detecting a position of a capsule endoscope in a subject, the capsule endoscope being introduced into the subject and moving in the subject, based on received strength of signals transmitted from the capsule endoscope at a plurality of receiving antennas. The program includes: region acquiring step of obtaining distances between receiving antennas and a capsule endoscope that is introduced into a subject and moves in the subject, based on received strength at the receiving antennas upon the receiving antennas receiving signals transmitted from the capsule endoscope, and acquiring a region where at least two spheres of a plurality of spheres with the receiving antennas as centers and the distances corresponding respectively to the receiving antennas as radii overlap with one another; estimating step of estimating orientations of the capsule endoscope at a plurality of points in the region based on positional relationships between the points and the receiving antennas, and the received strength of the signals received by the receiving antennas; and position determining step of determining a position of the capsule endoscope in the subject based on the positions of the points and the orientations of the capsule endoscope.

Advantageous Effects of Invention

According to the present invention, the orientation of the capsule endoscope is estimated, and the position of the capsule endoscope is further narrowed down with the orientation from a region acquired based on the received strength at each receiving antenna and accordingly it becomes possible to detect the position of the capsule endoscope with higher accuracy than before while cutting the calculation amount.

Brief Description of Drawings

FIG. 1 is a schematic diagram illustrating a configuration of a capsule endoscope system according to a first embodiment of the present invention.
FIG. 2 is a schematic diagram illustrating an internal configuration of a capsule endoscope illustrated in FIG. 1.
FIG. 3 is a block diagram illustrating a schematic configuration of an information processing device illustrated in FIG. 1.
FIG. 4 is a flowchart illustrating the operation of the information processing device illustrated in FIG. 1.
FIG. 5 is a flowchart illustrating the details of a position determination process illustrated in FIG. 4.
FIG. 6 is a schematic diagram illustrating a process of acquiring an existing region.
FIG. 7 is an enlarged view of the existing region illustrated in FIG. 6.
FIG. 8 is a flowchart illustrating the details of a process of estimating the orientation of the capsule endoscope.
FIG. 9 is a schematic diagram illustrating the process of estimating the orientation of the capsule endoscope.
FIG. 10 is a schematic diagram illustrating the process of estimating the orientation of the capsule endoscope.
FIG. 11 is a diagram illustrating a calculation principle of a theoretical value of received strength at each receiving antenna.
FIG. 12 is a diagram illustrating the calculation principle of the theoretical value of the received strength at each receiving antenna.

FIG. 13 is a diagram illustrating the calculation principle of the theoretical value of the received strength at each receiving antenna.

FIG. 14 is a diagram illustrating the calculation principle of the theoretical value of the received strength at each receiving antenna.

FIG. 15 is a diagram illustrating a process of correcting the moving locus of the capsule endoscope.

FIG. 16 is a diagram illustrating the process of correcting the moving locus of the capsule endoscope.

FIG. 17 is a schematic diagram illustrating another method for acquiring the existing region of the capsule endoscope.

FIG. 18 is a schematic diagram illustrating another configuration example of the capsule endoscope system according to the first embodiment of the present invention.

FIG. 19 is a block diagram illustrating a configuration example of a receiving device included in a capsule endoscope system according to a second embodiment of the present invention.

Description of Embodiments

Hereinafter, a description will be given of a position detection device, a capsule endoscope system, and a computer readable recording medium according to embodiments of the present invention with reference to the drawings. In the following description, a capsule endoscope system including a capsule endoscope that is inserted into the body of a subject and captures an in-vivo image of the subject is illustrated as an example of the position detection device and the capsule endoscope system according to the present invention. However, the embodiments shall not limit the present invention.

(First Embodiment)

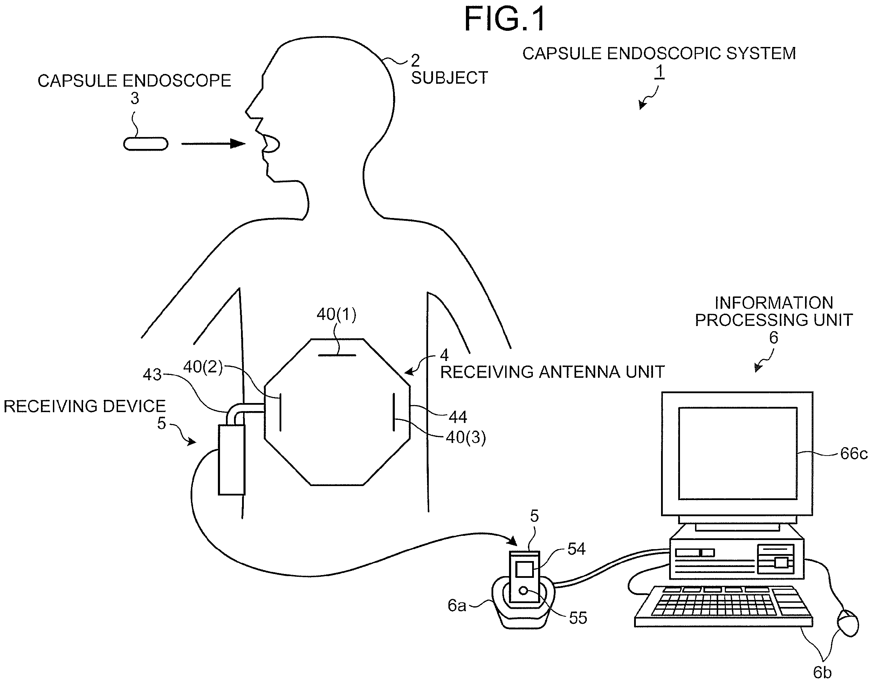

FIG. 1 is a schematic diagram illustrating a configuration of a capsule endoscope system 1 according to a first embodiment of the present invention. As illustrated in FIG. 1, the capsule endoscope system 1 includes a capsule endoscope 3 that captures an in-vivo image of a subject 2, a receiving device 5 that receives via a receiving antenna unit 4 a wireless signal transmitted wirelessly from the capsule endoscope 3 introduced into the subject 2, and an information processing device 6 that estimates an imaging position of an image obtained by capturing the inside of the subject 2 by the capsule endoscope 3 and displays an image of the inside of the subject 2 based on the image data obtained by being captured by the capsule endoscope 3.

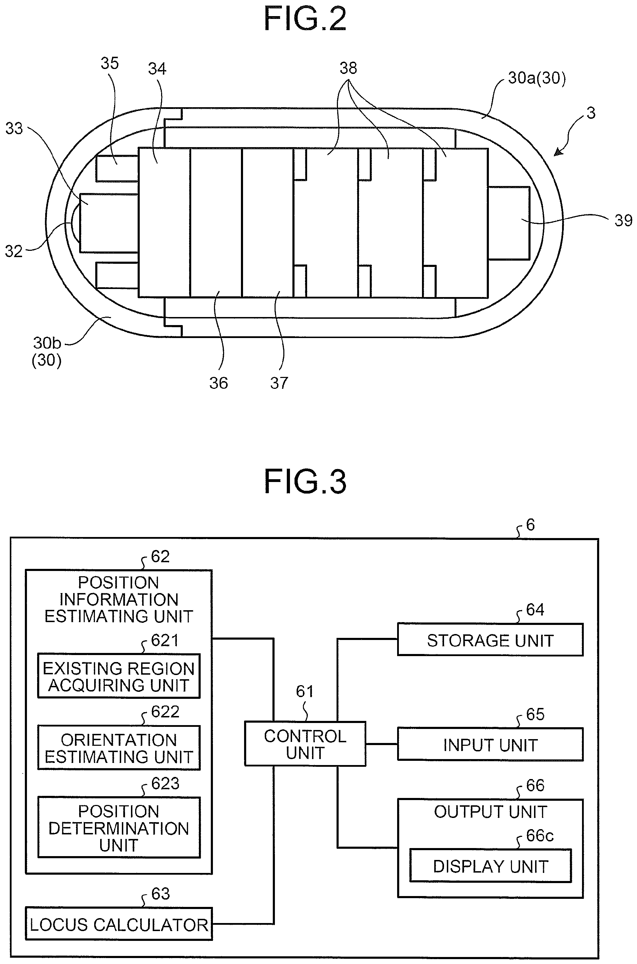

FIG. 2 is a schematic diagram illustrating an internal configuration of the capsule endoscope 3. As illustrated in FIG. 2, the capsule endoscope 3 is housed in a capsule-shaped container 30 (casing) including a substantially cylindrical shaped or a semi-elliptical shaped container 30a forming a hemispherical dome at one end, and opening at the other end, and a hemispherical optical dome 30b that is inserted into an opening of the container 30a to make the container 30a watertight. The capsule-shaped container 30 (30a and 30b) is, for example, the size to the degree that the subject 2 can swallow. Moreover, in the first embodiment, at least the optical dome 30b is formed with transparent material.

The capsule endoscope 3 includes an objective lens 32 that forms an image with light incident through the optical dome 30b, a lens frame 33 to which the objective lens 32 is attached, an imaging unit 34 that converts an optical signal incident from the objective lens 32 into an electrical signal and forms an imaging signal, a lighting unit 35 that illuminates the inside of the subject 2 upon image capture, a circuit board 36 where a processing circuit that drives the imaging unit 34 and the lighting unit 35 and generates an image signal from the imaging signal input from the imaging unit 34, and the like are formed, a transmitting/receiving circuit 37 that transmits the image signal and receives signals from the receiving device 5 and the like outside the body cavity, a plurality of button cells 38 that supplies power to these function units, and an antenna 39 in, for example, a circular coil form or circular loop form.

After being swallowed by the subject 2, the capsule endoscope 3 passes through the esophagus in the subject 2 and moves in the body cavity by the peristaltic movement of the lumen of the digestive tract. The capsule endoscope 3 consecutively captures the inside of the body cavity of the subject 2 at minute time intervals, for example, at 0.5-second intervals while moving in the body cavity, and generates image data to sequentially transmit the image data to the receiving device 5. In the first embodiment, it is also possible to execute a position estimation process by an image signal of image data generated by the imaging unit 34 of the capsule endoscope 3 capturing an image. However, it is more preferred to generate a transmission signal including the generated image signal and a received strength detection signal for detecting the position of the capsule endoscope 3 and execute a position detection process by the received strength detection signal that is easy to detect received strength.

The receiving device 5 is connected by the sheet-shaped receiving antenna unit 4 where a plurality of (three in FIG. 1) receiving antennas 40(1) to 40(3) are arranged, and an antenna cable 43. The receiving device 5 receives wireless signals transmitted from the capsule endoscope 3 respectively via the receiving antennas 40(1) to 40(3) and detects the received field strength of the wireless signal for each of the receiving antennas 40(1) to 40(3) as well as acquiring image data of the inside of the subject 2 based on the received wireless signals. The receiving device 5 associates received field strength information of the receiving antennas 40(1) to 40(3), time information indicating a time, and the like with the received image data and stores the image data in a storage unit to be described below. Moreover, the receiving device 5 may include a display unit 54 that displays an image corresponding to image data received from the capsule endoscope 3, and an operating unit 55 used when an instruction operation on the receiving device 5 is input.

It is preferred that the receiving antennas 40(1) to 40(3) be antennas using only main polarization (in other words, not using cross polarization). In the embodiment, dipole antennas are used as the receiving antennas 40(1) to 40(3). Hereinafter, these receiving antennas 40(1) to 40(3) are also described as a receiving antenna 40(n) (n = 1 to N, N = 3 in FIG. 1).

The receiving device 5 is carried by the subject 2 while the capsule endoscope 3 is capturing images, in other words, during a period of time from when the capsule endoscope 3 is inserted, for example, from the mouth of the subject 2 to when it passes through the digestive tract and is discharged from the subject 2. The receiving device 5 is removed from the subject 2 after the end of the examination with the capsule endoscope 3, and is connected to the information processing device 6 to transfer information on the image data received from the capsule endoscope 3, and the like.

The receiving antennas 40(n) are arranged in predetermined positions of a sheet 44, for example, positions corresponding to the organs in the subject 2, the organs being a passage route of the capsule endoscope 3, when the receiving antenna unit 4 is attached to the subject 2. The arrangement of the receiving antennas 40(n) can be freely changed according to the purpose such as an examination or diagnosis.

The information processing device 6 is configured using a work station or a personal computer including a display unit 66c of a liquid crystal display or the like. The information processing device 6 is an image display device that displays an image corresponding to image data of the inside of the subject 2 acquired via the receiving device 5 and is also a position detection device that detects the position of the capsule endoscope 3 when the image was captured.

The information processing device 6 is connected to a cradle 6a that reads image data and the like from the storage unit of the receiving device 5 and an operation input device 6b such as a keyboard and a mouse. The cradle 6a acquires image data and associated information associated with the image data, such as received field strength information, time information, and identification information of the capsule endoscope 3, from a memory of the receiving device 5 when the receiving device 5 is worn, and transfers the acquired various pieces of information to the information processing device 6. The operation input device 6b accepts input by a user. Consequently, the user observes living body parts in the subject 2, for example, esophagus, stomach, small intestine, and large intestine while operating the operation input device 6b and watching images of the inside of the subject 2 that are sequentially displayed by the information processing device 6, and diagnoses the subject 2.

Next, a configuration of the information processing device 6 illustrated in FIG. 1 will be described in detail. FIG. 3 is a block diagram illustrating the configuration of the information processing device 6 illustrated in FIG. 1. As illustrated in FIG. 3, the information processing device 6 includes a control unit 61 that performs centralized control of the entire information processing device 6, a position information estimating unit 62 that estimates the position of the capsule endoscope 3 at a timing when a wireless signal including image data is received, and generates position information, a locus calculator 63 that calculates the moving locus of the capsule endoscope 3 in the subject 2 based on the position information of the capsule endoscope 3 generated by the position information estimating unit 62 on the basis of each image data, a storage unit 64 that stores image data received from the capsule endoscope 3 and signal strength, an input unit 65 that acquires information from the operation input device 6b such as a keyboard and a mouse, and the like, and an output unit 66 that includes the display unit 66c configured by a display and is configured using a printer, a speaker, and the like. The storage unit 64 is configured using a hard disk that magnetically stores information, and a memory that loads from the hard disk various programs, parameters, and the like that are related to a process when the capsule endoscope system 1 executes the process, and electrically stores them.

The position information estimating unit 62 acquires the strength of signals respectively received by the plurality of receiving antennas 40(n) of the receiving antenna unit 4 (the received field strength, hereinafter also simply referred to as the received strength), and estimates the position of the capsule endoscope 3 (the position of the built-in antenna 39) from the strength of these signals. The position information estimating unit 62 includes an existing region acquiring unit 621, an orientation estimating unit 622, and a position determination unit 623.

The existing region acquiring unit 621 estimates the existing region of the capsule endoscope 3 at a position detection timing based on the received strength at the receiving antennas 40(n).

The orientation estimating unit 622 estimates the orientation of the capsule endoscope 3, in other words, the orientation of the antenna 39 contained in the capsule endoscope 3 based on positional relationships between a plurality of points in the existing region and the receiving antennas 40 (n), and the received strength at the receiving antennas 40(n) when the capsule endoscope 3 is assumed to exist at each of the plurality of points.

The position determination unit 623 detects a more detailed position of the capsule endoscope 3 in the existing region based on the positions of the points in the existing region, and the orientations of the capsule endoscope 3 estimated by the orientation estimating unit 622.

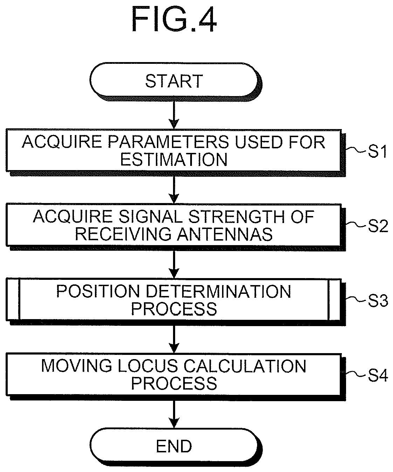

Next, the operation of the information processing device 6 illustrated in FIG. 1 will be described. FIG. 4 is a flowchart illustrating the operation of the information processing device 6.

Firstly, in Step S1, the position information estimating unit 62 acquires from the storage unit 64 parameters used to estimate the position of the capsule endoscope 3. The content of the parameters is described below.

In the following Step S2, the information processing device 6 acquires the signal strength of a signal received by each receiving antenna 40(n) at the position detection timing.

In the following Step S3, the position information estimating unit 62 determines the position of the capsule endoscope 3 in the subject 2 based on the received strength at each receiving antenna 40(n), and creates position information.

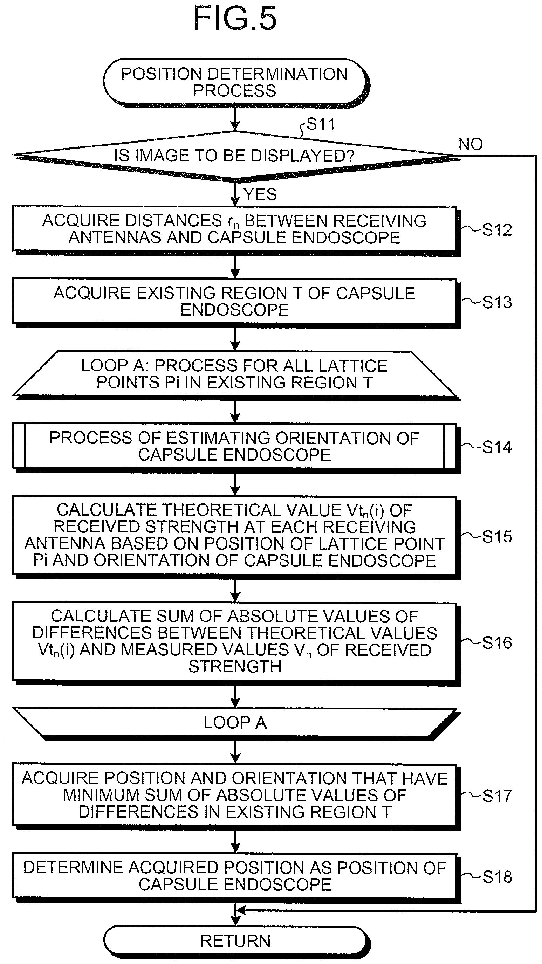

FIG. 5 is a flowchart illustrating the details of a position determination process executed by the position information estimating unit 62.

Firstly, in Step S11, the position information estimating unit 62 judges whether or not an image corresponding to image data input into the information processing device 6 is an image to be displayed on the display unit 66c. For example, if the ratio of noise included in image data is larger than a predetermined threshold value, it is judged that an image corresponding to the image data is not an image to be displayed on the display unit 66c. If an image corresponding to image data input is not an image to be displayed on the display unit 66c (Step S11: No), the processing returns to the main routine. This is because such an image is judged to be an image unsuitable for the user to observe.

If an image corresponding to image data input is an image to be displayed on the display unit 66c (Step S11: Yes), the existing region acquiring unit 621 acquires distances $r_n$ between the receiving antennas 40(n) and the capsule endoscope 3 from the received strength at the receiving antennas 40n (Step S12).

In more detail, the existing region acquiring unit 621 obtains voltage $V_n$ of the received signal based on the received field strength at the receiving antenna 40(n), and calculates the distance $r_n$ using the following equation (1).

$$V_n = K \frac{1}{r_n} e^{-\alpha r_n} \quad \dots \quad (1)$$

In equation (1), the parameter K is a constant determined by the characteristic of the receiving antenna 40(n), and the parameter $\alpha$ is an attenuation coefficient of biological tissue. These parameters K and $\alpha$ are derived from actual measured values previously measured, and acquired from the storage unit 64 in Step S1.

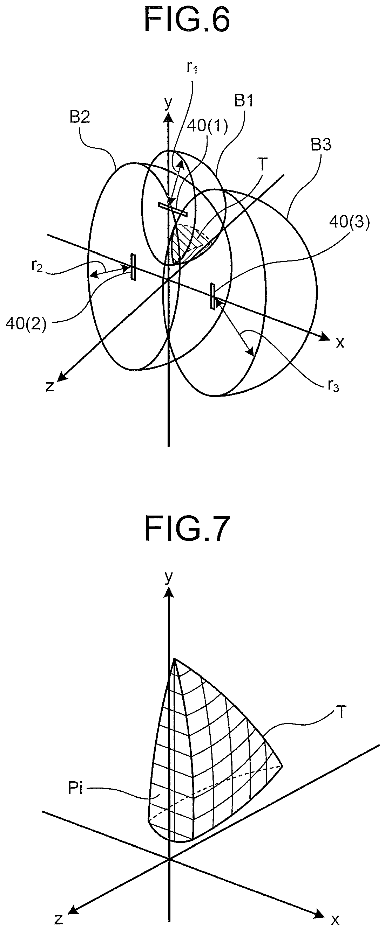

In the following Step S13, the existing region acquiring unit 621 acquires a region where the capsule endoscope 3 exists (existing region) T based on the distances $r_n$ between the receiving antennas 40(n) and the capsule endoscope 3. In more detail, a region where at least two or more spheres of a plurality of spheres with the receiving antennas 40(n) as centers and the distances $r_n$ as radii overlap with one another is acquired as the existing region T. For example, in a case of the receiving antennas 40(1) to 40(3), as illustrated in FIG. 6, a region where a sphere B1 with the receiving antenna 40(1) as a center and a distance $r_1$ as a radius, a sphere B2 with the receiving antenna 40(2) as a center and a distance $r_2$ as a radius, and a sphere B3 with the receiving antenna 40(3) as a center and a distance $r_3$ as a radius overlap with one another is acquired as the existing region T of the capsule endoscope 3. If the coordinates of the receiving antennas 40(1) to 40(3) are taken on such a X-Y plane as illustrated in FIG. 6, the spheres B1 to B3 where the capsule endoscope 3 is estimated to be located can be considered to be, for example, hemispheres on the $Z \leq 0$ side (the subject 2 side).

FIG. 7 is an enlarged view of the existing region T illustrated in FIG. 6. Following Step S13, the position information estimating unit 62 executes a process of a loop A on points (for example, center points. Hereinafter referred to as the lattice points) Pi in a plurality of sub-regions obtained by dividing the existing region T in a lattice form.

In Step S14, the orientation estimating unit 622 executes a process of estimating the orientation of the capsule endoscope 3 when the capsule endoscope 3 is assumed to exist at the lattice point Pi.

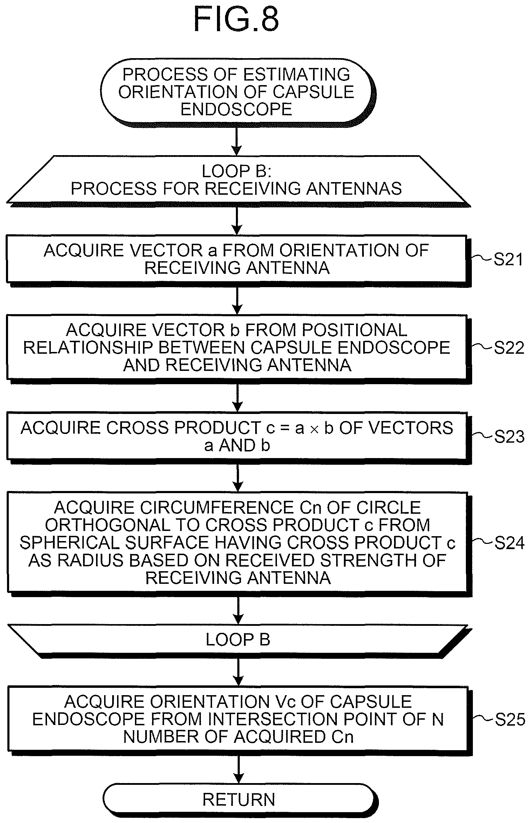

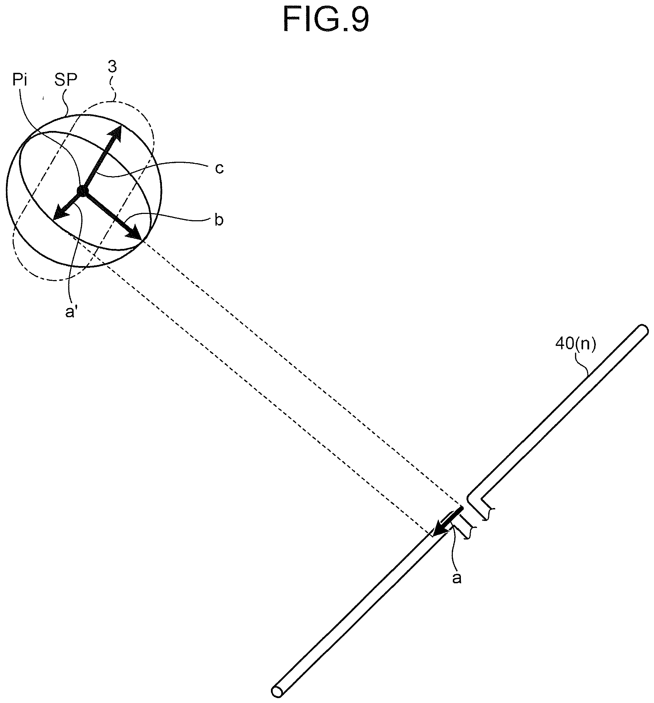

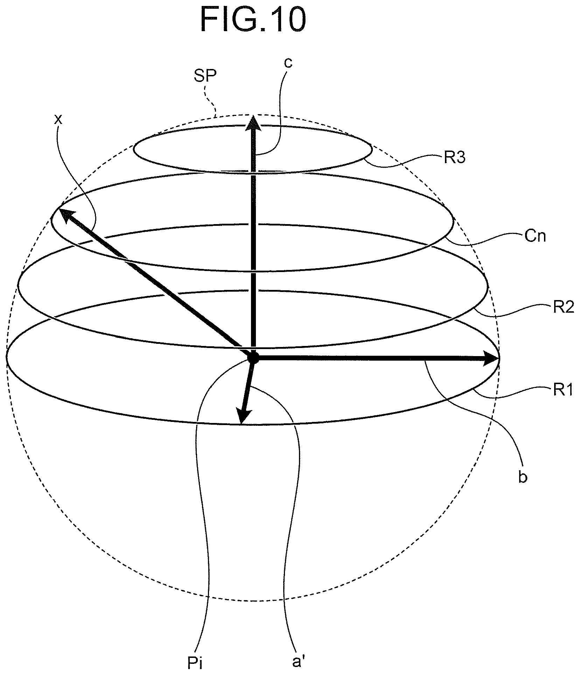

FIG. 8 is a flowchart illustrating the details of the process of estimating the orientation of the capsule endoscope 3, the process being executed by the orientation estimating unit 622. Moreover, FIGS. 9 and 10 are schematic diagrams illustrating the process of estimating the orientation of the capsule endoscope 3.

As illustrated in FIG. 8, the orientation estimating unit 622 executes a process of a loop B on each of the receiving antennas 40(n) (n = 1 to N, n is a natural number).

Firstly, in Step S21, the orientation estimating unit 622 acquires a vector a that represents the orientation of the receiving antenna 40(n) being a process target as illustrated in FIG. 9. FIG. 9 also illustrates, as a vector a', a vector that is the vector a moved parallel to the center (in other words, the lattice point Pi) of the capsule endoscope 3.

In the following Step S22, the orientation estimating unit 622 acquires a vector b pointing to the receiving antenna 40(n) from the capsule endoscope 3 (the lattice point Pi) based on the positional relationship between the capsule endoscope 3 and the receiving antenna 40(n).

In the following Step S23, the orientation estimating unit 622 acquires a vector c being the cross product of the vector a (in other words, the vector a') and the vector b (c = a x b).

Furthermore, in Step S24, the orientation estimating unit 622 acquires a circumference Cn of a circle orthogonal to the vector c based on a spherical surface SP having the vector c as a radius based on the received strength at the receiving antenna 40(n). As illustrated in FIG. 10, the orientation of the capsule endoscope 3 (in other words, the orientation of the antenna 39 contained in the capsule endoscope 3) can be expressed as a vector starting from the same starting point (the lattice point Pi) as the vector c and having any point on the spherical surface SP as an endpoint (hereinafter referred to as a vector x). At this point, in terms of the circumference of the circle orthogonal to the vector c (for example, circumferences R1, R2, ..., and so forth), even if the vector x passes at any point on one circumference, a theoretical value of the received strength at the antenna 40(n) does not change. Hence, the orientation estimating unit 622 acquires one circumference Cn orthogonal to the vector c as a set of endpoint candidates of the vector x based on the received strength of the antenna 40(n).

After the circumferences Cn are acquired for all (N number) of the receiving antennas 40(n), in Step S25, the orientation estimating unit 622 obtains an intersection point of N number of the circumferences Cn, and acquires an orientation Vc of the capsule endoscope 3 (the antenna 39) from the intersection point. Afterwards, the processing returns to the main routine.

In Step S15 (see FIG. 5) following Step S14, the position determination unit 623 calculates a theoretical value $Vt_n(i)$ of the received strength at each of the receiving antennas 40(n) from the position of the lattice point Pi and the orientation Vc of the capsule endoscope 3.

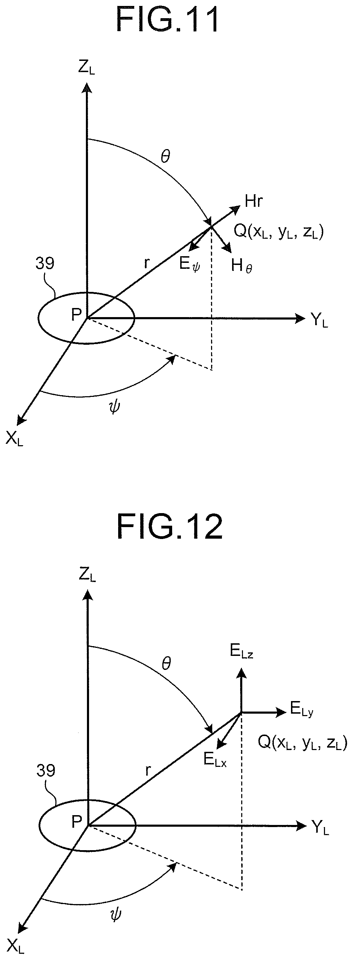

As illustrated in FIG. 11, in a coordinate system $X_L Y_L Z_L$ (an origin P) relative to the circular coil or circular loop shaped antenna 39 placed in the capsule endoscope 3, magnetic-field components $H_r$ and $H_\theta$ of a magnetic field (including components of an electrostatic field, a radiation field, and an induction filed), and an electric field component $E_\psi$ at an arbitrary point Q $(x_L, y_L, z_L)$ are expressed by the following equations (2-1) to (2-3).

$$H_r = \frac{IS}{2\pi}\left(\frac{jk}{r^2} + \frac{1}{r^3}\right) \cdot e^{-jkr} \cdot \cos\theta \ \ldots \ (2-1)$$

$$H_\theta = \frac{IS}{4\pi}\left(-\frac{k^2}{r} + \frac{jk}{r^2} + \frac{1}{r^3}\right) \cdot e^{-jkr} \cdot \sin\theta \ \ldots \ (2-2)$$

$$E_\phi = -\frac{j\omega\mu IS}{4\pi}\left(\frac{jk}{r} + \frac{1}{r^2}\right) \cdot e^{-jkr} \cdot \sin\theta \ \ldots \ (2-3)$$

In equations (2-1) to (2-3), the symbol I denotes electric current flowing through the antenna 39, and the symbol S denotes the area of the circular coil constructing the antenna 39. The symbol r denotes a distance between the antenna 39 and an arbitrary position ($r = (x^2 + y^2 + z^2)^{1/2}$). Moreover, $k = \omega(\varepsilon\mu)^{1/2}$ (where $\varepsilon$ is a dielectric constant and $\mu$ is magnetic permeability), and the symbol j denotes an imaginary unit.

If a frequency of a magnetic field generated by the antenna 39 placed in the capsule endoscope 3 is high, and the capsule endoscope 3 is sufficiently apart from the receiving antenna 40(n) attached to the body surface of the subject 2, the radiation field component is the largest in the magnetic field (electromagnetic wave) reaching each of the receiving antennas 40(n). Therefore, the electrostatic field and induction field components are smaller than the radiation field component and accordingly, they can be ignored. Hence, equations (2-1) to (2-3) can be modified as in the following equations (3-1) to (3-3).

$$H_r = 0 \ \ldots \ (3\text{-}1)$$

$$H_\theta = \frac{IS}{4\pi}\left(-\frac{k^2}{r}\right)\cdot e^{-jkr}\cdot\sin\theta \qquad \dots (3\text{-}2)$$

$$E_\phi = -\frac{j\omega\mu IS}{4\pi}\left(\frac{jk}{r}\right)\cdot e^{-jkr}\cdot\sin\theta \qquad \dots (3\text{-}3)$$

Assuming that the receiving antennas 40(n) attached to the body surface of the subject 2 are electric-field detection antennas for detecting an electric field, an equation necessary for the detection is equation (3-3). The electric field component $E_\psi$ given by equation (3-3) denotes a radiation electric field, and is considered to be the result by the AC theory. Therefore, an instantaneous value of the electric field component $E_\psi$ is obtained by multiplying both sides of equation (3-3) by $\exp(j\omega t)$ as in the following equation (4), and extracting a real part.

$$E_\phi e^{j\omega t} = -\frac{j\omega\mu IS}{4\pi}\cdot\frac{jk}{r}\cdot e^{-jkr}\cdot\sin\theta\cdot e^{j\omega t}$$

$$= \frac{\omega\mu ISk}{4\pi r}(\cos U + j\sin U)\cdot\sin\theta \qquad \dots (4)$$

In equation (4), $U = \omega t - kr$.
If the real part of equation (4) is extracted, an instantaneous value $E'_\psi$ of the electric field is given by the following equation (5).

$$E'_\phi = \frac{\omega\mu ISk}{4\pi r}\cos U\cdot\sin\theta \qquad \dots (5)$$

Moreover, if equation (4) is converted from a polar coordinate system (r, $\theta$, $\psi$) into an orthogonal coordinate system ($X_L$, $Y_L$, $Z_L$) as illustrated in FIG. 12, coordinate components ($E_{Lx}$, $E_{Ly}$, $E_{Lz}$) of the instantaneous value $E'_\psi$ of the electric field are given by the following equations (6-1) to (6-3).

$$E_{Lx} = E'_\phi\cdot\sin\phi = \frac{\omega\mu ISk}{4\pi r^2}\cdot\cos U\cdot(-y_L)\dots \quad (6\text{-}1)$$

$$E_{Ly} = E'_\phi\cdot\cos\phi = \frac{\omega\mu ISk}{4\pi r^2}\cdot\cos U\cdot x_L\dots \quad (6\text{-}2)$$

$$E_{Lz} = 0\dots \quad (6\text{-}3)$$

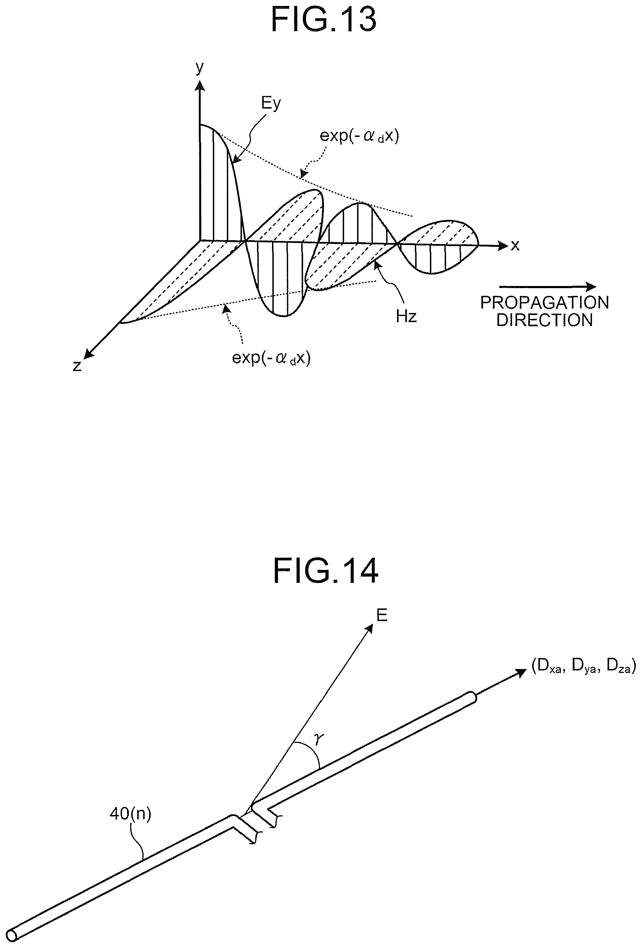

As illustrated in FIG. 13, if electromagnetic waves $E_y$ and $H_z$ propagate through a medium, the energy of the electromagnetic waves is absorbed in the medium in accordance with the characteristics of the medium such as electrical conductivity. For example, energy $A_r$ of the electromagnetic waves $E_y$ and $H_z$ propagating in the x direction is exponentially attenuated by a damping factor $\alpha_d$ as expressed by the following equations (7) and (8).

$$A_r = e^{-\alpha_d X} \quad \ldots \quad (7)$$

$$\alpha_d = \left( \frac{\omega^2 \varepsilon \mu}{2} \right)^{\frac{1}{2}} \left\{ \left( 1 + \frac{k^2}{\omega^2 \varepsilon^2} \right)^{\frac{1}{2}} - 1 \right\}^{\frac{1}{2}} \quad \ldots \quad (8)$$

In equation (8), $\varepsilon = \varepsilon_0 \varepsilon_r$ ($\varepsilon_0$: dielectric constant of a vacuum, $\varepsilon r$: dielectric constant of a medium), $\mu = \mu_0 \mu_r$ ($\mu_0$: magnetic permeability of a vacuum, $\mu_r$: magnetic permeability of a medium), $\omega$ is an angular frequency, k is electric conductivity.

Therefore, an instantaneous value $E_L$ of an electric field when the characteristics in the living body are considered is given by the following equations (9-1) to (9-3).

$$E_{Lx} = A_r E'_\phi \sin \phi = e^{-\alpha_d r} \cdot \frac{\omega \mu I S k}{4 \pi r^2} \cdot \cos U \cdot (-y_L) \quad \ldots \quad (9\text{-}1)$$

$$E_{Ly} = A_r E'_\phi \cos \phi = e^{-\alpha_d r} \cdot \frac{\omega \mu I S k}{4 \pi r^2} \cdot \cos U \cdot x_L \quad \ldots \quad (9\text{-}2)$$

$$E_{Lz} = 0 \quad \ldots \quad (9\text{-}3)$$

Moreover, an equation that converts the position Q ($x_L$, $y_L$, $z_L$) in the coordinate system $X_L Y_L Z_L$ relative to the antenna 39 of the capsule endoscope 3 into a coordinate system $X_W Y_W Z_W$ relative to the subject 2 is as in the following equation (10).

$$\begin{pmatrix} x_{LP} \\ y_{LP} \\ z_{LP} \end{pmatrix} = R^{-1} \left[ \begin{pmatrix} x_{WP} \\ y_{WP} \\ z_{WP} \end{pmatrix} - \begin{pmatrix} x_{WG} \\ y_{WG} \\ z_{WG} \end{pmatrix} \right] = \begin{pmatrix} R_{00} & R_{01} & R_{02} \\ R_{10} & R_{11} & R_{12} \\ R_{20} & R_{21} & R_{22} \end{pmatrix} \left[ \begin{pmatrix} x_{WP} \\ y_{WP} \\ z_{WP} \end{pmatrix} - \begin{pmatrix} x_{WG} \\ y_{WG} \\ z_{WG} \end{pmatrix} \right] \quad \ldots \quad (10)$$

In equation (10), ($x_{WP}$, $y_{WP}$, $z_{WP}$) and ($x_{WG}$, $y_{WG}$, $z_{WG}$) represent the position Q and the position of the antenna 39 in the coordinate system $X_W Y_W Z_W$, respectively.

The matrix having, as components, $R_{00}$ to $R_{22}$ shown on the right side of equation (10) represents a rotation matrix of the coordinate system $X_W Y_W Z_W$ and the coordinate system $X_L Y_L Z_L$, and is given by the following equation (11).

$$\begin{pmatrix} R_{00} & R_{10} & R_{20} \\ R_{01} & R_{11} & R_{21} \\ R_{02} & R_{12} & R_{22} \end{pmatrix} = \begin{pmatrix} \cos \alpha \cos \beta & -\sin \alpha & \cos \alpha \sin \beta \\ \sin \alpha \cos \beta & \cos \alpha & \sin \alpha \sin \beta \\ -\sin \beta & 0 & \cos \beta \end{pmatrix} \quad \ldots \quad (11)$$

In equation (11), $\alpha$ and $\beta$ denote the rotation amounts in a polar coordinate system, respectively.

Consequently, an electric field $E_W$ at the position Q ($x_{WP}$, $y_{WP}$, $z_{WP}$) in the coordinate system $X_W Y_W Z_W$ relative to the subject 2 is given by the following equation (12).

$$\begin{pmatrix} E_{Wx} \\ E_{Wy} \\ E_{Wz} \end{pmatrix} = R \begin{pmatrix} E_{Lx} \\ E_{Ly} \\ E_{Lz} \end{pmatrix} = \begin{pmatrix} R_{00} & R_{10} & R_{20} \\ R_{01} & R_{11} & R_{21} \\ R_{02} & R_{12} & R_{22} \end{pmatrix} \begin{pmatrix} E_{Lx} \\ E_{Ly} \\ E_{Lz} \end{pmatrix} \dots \quad (12)$$

Equations (9-1) to (11) are substituted into equation (12) to obtain equation (13) that provides the electric field $E_W$.

$$\begin{pmatrix} E_{Wx} \\ E_{Wy} \\ E_{Wz} \end{pmatrix} = \frac{k_1}{r^2} e^{-\alpha_d r} \begin{pmatrix} 0 & (z_{WP} - z_{WG}) & -(y_{WP} - y_{WG}) \\ (z_{WP} - z_{WG}) & 0 & (x_{WP} - x_{WG}) \\ (y_{WP} - y_{WG}) & -(x_{WP} - x_{WG}) & 0 \end{pmatrix} \begin{pmatrix} g_x \\ g_y \\ g_z \end{pmatrix} \dots \quad (13)$$

In equation (13), $k_1$ is a constant. Moreover, $(g_x, g_y, g_z)$ represents the orientation of the antenna 39 acquired in Step S14.

Therefore, the theoretical value $Vt_n(i)$ of the received strength detected when the receiving antenna 40(n) constructed of a dipole antenna receives the electric field $E_W$ generated from the antenna 39 is given by the following equation (14).

$$Vt_n(i) = k_2 E_W \cos\gamma$$
$$= k_2 (E_{Wx} D_{xa} + E_{Wy} D_{ya} + E_{Wz} D_{za}) \dots \quad (14)$$

In equation (14), $k_2$ is a constant. Moreover, $(D_{xa}, D_{ya}, D_{za})$ represents the orientation of the receiving antenna 40(n) in a coordinate system relative to the subject 2.

In the following Step S16, the position determination unit 623 calculates the sum $M(i)$ of the absolute values of differences between the theoretical values $Vt_n(i)$ of the received strength of the receiving antennas 40(n) calculated in Step S15 and actual received strength (measured values) $V_n$ at the antennas 40(n) by the following equation (15).

$$M(i) = \sum_{n=1}^{N} |Vt_n(i) - V_n| \dots \quad (15)$$

In the first embodiment, N = 3.

The process of the loop A is repeated for all the lattice points Pi, and then in Step S17, the position determination unit 623 acquires the lattice point Pi having the minimum sum $M(i)$ of the absolute values of the differences in the existing region T and the orientation Vc of the capsule endoscope 3 at that point in time.

Furthermore, in Step S18, the position determination unit 623 determines the position of the acquired lattice point Pi as the position of the capsule endoscope 3. At this point, the position determination unit 623 creates position information representing the determined position and stores the position information in the storage unit 64. Afterwards, the processing returns to the main routine.

In Step S4 (see FIG. 4) following Step S3, the locus calculator 63 executes a moving locus calculation process for calculating the moving locus of the capsule endoscope 3 in the subject 2 by connecting temporally neighboring positions of the capsule endoscope 3 based on the position information of the capsule endoscope 3 created by the position information estimating unit 62 and the position information of the capsule endoscope 3 hitherto stored in the storage unit 64.

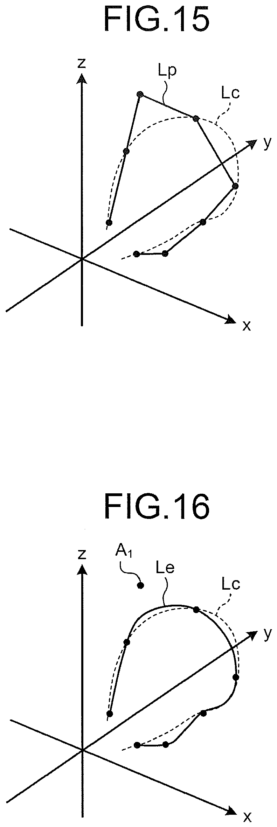

The detected position of the capsule endoscope 3 may contain an error due to an error of arrangement of the receiving antennas 40(n), noise, and the like. For example, as illustrated in FIG. 15, a moving locus Lp linking the detected positions of the capsule endoscope 3 may deviate from an actual moving locus Lc of the capsule endoscope 3. However, the capsule endoscope 3 moves in the organs in the subject 2 and accordingly it is considered not to move greatly in a short time in reality.

Hence, the locus calculator 63 calculates the locus while performing a correction process such a median filtering process that obtains a median value from temporally neighboring coordinates (for example, three coordinates including the previous and subsequent ones). As a consequence, as illustrated in FIG. 16, the moving locust Lc that is closer to the actual moving locus Lp can be acquired. The moving locus Lc is one where the influence of a detected position $A_1$, which deviated greatly from the actual moving locus Lp due to an error of the arrangement of the receiving antennas 40(n), noise, and the like, has been reduced. Moreover, the correction process is not limited to the median filtering process, but may also use a moving average process for obtaining an average value of, for example, five coordinates including the two previous and following coordinates. The calculated moving locus is displayed on the display unit 66c together with an image where the inside of the subject 2 appears and is stored in the storage unit 64.

As described above, according to the first embodiment, the orientation of the capsule endoscope 3 is estimated, and the position of the capsule endoscope 3 is further narrowed down with the orientation from the existing region acquired based on the received strength at each of the receiving antennas 40(n). Accordingly, it becomes possible to perform position detection with higher accuracy than before.

Moreover, according to the first embodiment, a complicated calculation process becomes unnecessary and accordingly it becomes possible to enhance the speed of the position determination process and the moving locus calculation process.

Moreover, according to the first embodiment, the sheet-shaped receiving antenna unit 4 where the plurality of receiving antennas 40(n) is arranged is used. Accordingly, there is no need to adjust the arrangement of the receiving antennas 40(n) on every examination. Furthermore, the receiving antenna unit 4 where the arrangement of the receiving antennas 40(n) is determined in advance is used. Therefore, it is also possible to avoid a problem that the accuracy of the process of estimating the position of the capsule endoscope 3 is reduced with displacements of the receiving antennas 40(n).

In the first embodiment, when the position of the capsule endoscope 3 is determined, the sum of the absolute values of differences between theoretical values and measured values of received strength at the receiving antennas 40(n) is calculated. However, as long as a minimum value of an error between a theoretical value and a measured value can be found, another computation method may be used. For example, the sum of squared residuals of a theoretical value and a measured value may be calculated to detect the lattice point Pi having the minimum sum of squared residuals.

Moreover, in the first embodiment, a region where all spheres with three receiving antennas 40(n) as their centers and the distances $r_n$ corresponding respectively to the receiving antennas 40(n) as radii overlap with one another is acquired as the existing region of the capsule endoscope 3 (see FIG. 7). However, the capsule endoscope 3 is definitely located inside a region where at least two or more spheres overlap with one another. Hence, the existing region acquiring unit 621 may acquire, as the existing region of the capsule endoscope 3, a region where, for example, two spheres corresponding to two receiving antennas 40(n) of three receiving antennas 40(n) overlap with each other.

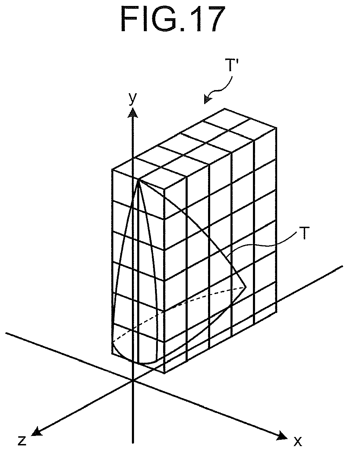

Moreover, in the first embodiment, the existing region T is acquired based on the receiving antennas 40(n) at every position detection timing. However, the capsule endoscope 3 is considered not to move greatly in a short time in reality so that the existing region T this time may be estimated based on the existing region T acquired last time. For example, as illustrated in FIG. 17, if the existing region T of the capsule endoscope 3 was acquired in the last position determination process, the process of the loop A (see FIG. 5) may be executed next time on the inside of a region T where an area where the capsule endoscope 3 can move is added to the existing region T.

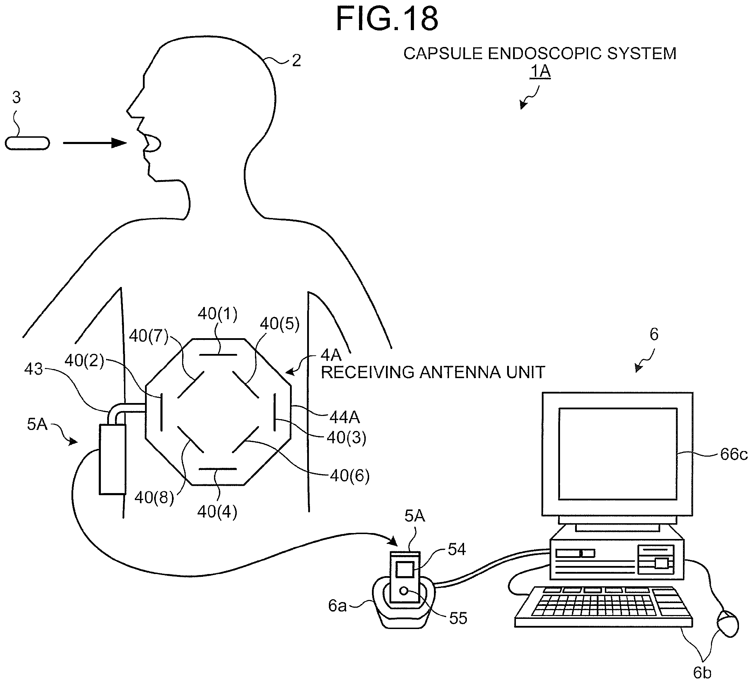

Moreover, in the first embodiment, the example where three receiving antennas 40(n) are provided to the receiving antenna unit 4 is described. However, there is no need to interpret the number of the receiving antennas limiting it to three. For example, a sheet 44A where eight receiving antennas 40(1) to 40(8) are arranged may be used as in a receiving antenna unit 4A connected to a receiving device 5A of a capsule endoscope system 1A illustrated in FIG. 18. Moreover, the arrangement of a plurality of the receiving antennas 40(n) may be optionally changed according to the purpose such as an examination or diagnosis.

(Second Embodiment)

Next, a second embodiment of the present invention will be described.

In the first embodiment, the information processing device 6 including the position information estimating unit 62 and the locus calculator 63 estimates the position of the capsule endoscope 3 and calculates the locus. However, it may be configured such that an estimating unit that estimates position information and a locus calculator are provided on a receiving device side, and the position of the capsule endoscope 3 is estimated at a timing when the inside of the subject 2 is captured on the receiving device side. In this case, the receiving device functions as the position detection device. In the second embodiment, a configuration of the receiving device in this case will be described.

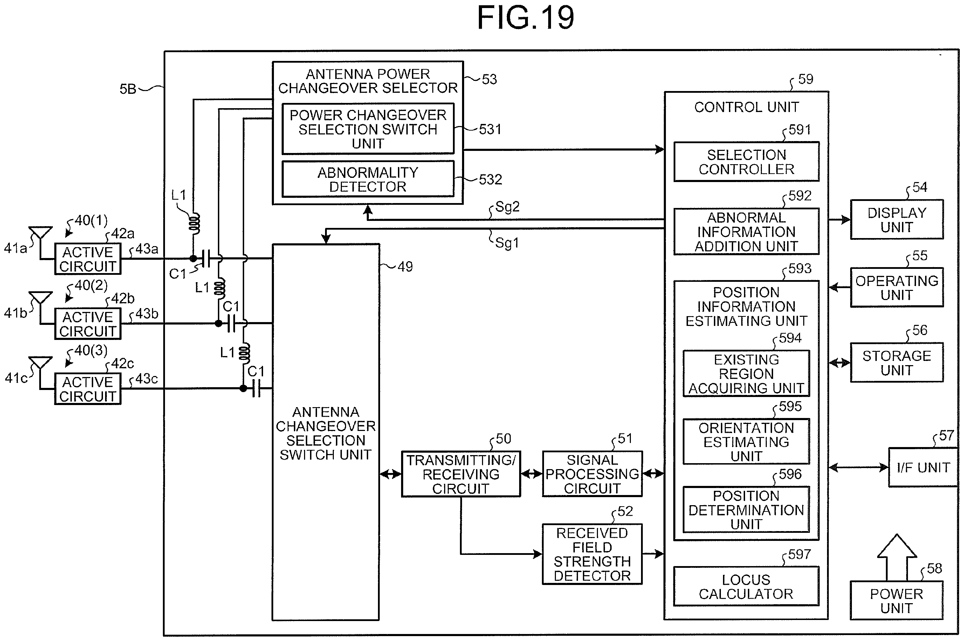

FIG. 19 is a block diagram illustrating a configuration example of a receiving device included in a capsule endoscope system according to the second embodiment. As illustrated in FIG. 19, a receiving device 5B includes the receiving antennas 40(n) (n = 1 to 3), an antenna changeover selection switch unit 49 that alternatively switches between the receiving antennas 40(n), a transmitting/receiving circuit 50 that executes processes such as demodulation on a wireless signal received via the receiving antenna 40(n) selected by the antenna changeover selection switch unit 49, a signal processing circuit 51 that executes signal processing for extracting image data and the like from a wireless signal output from the transmitting/receiving circuit 50, a received field strength detector 52 that detects the received field strength based on the strength of the wireless signal output from the transmitting/receiving circuit 50, an antenna power changeover selector 53 that alternatively switches between the receiving antennas 40(1) to 40(3) and supplies electric power to any of the receiving antennas 40(1) to 40(3), the display unit 54 that displays an image corresponding to image data received from the capsule endoscope 3, the operating unit 55 used when an instruction operation on the receiving device 5B is input, a storage unit 56 that stores various pieces of information including image data received from the capsule endoscope 3, an I/F unit 57 that transmits and receives to and from an information processing device in a mutual direction via a cradle 6a, a power unit 58 that supplies electric power to the units of the receiving device 5B, and a control unit 59 that controls the operation of the receiving device 5B. Of them, the control unit 59 includes a position information estimating unit 593 having a similar function to that of the position information estimating unit 62 illustrated in FIG. 3, and a locus calculator 597 having a similar function to that of the locus calculator 63.

The receiving antenna 40(1) includes an antenna unit 41a, an active circuit 42a, and an antenna cable 43a. The antenna unit 41a is configured using, for example, a dipole antenna, and receives a wireless signal transmitted from the capsule endoscope 3. The active circuit 42a is connected to the antenna unit 41a, and performs such things as impedance matching of the antenna unit 41a, and amplification and attenuation of a received wireless signal. The antenna cable 43a is configured using a coaxial cable, is connected at one end to the active circuit 42a, and is electrically connected at the other end to the antenna changeover selection switch unit 49 and the antenna power changeover selector 53 of the receiving device 5B. The antenna cable 43a transmits a wireless signal received by the antenna unit 41a to the receiving device 5B and transmits electric power supplied from the receiving device 5B to the active circuit 42a. The receiving antennas 40(2) and 40(3) have a similar configuration to that of the receiving antenna 40(1). Accordingly, the description will be omitted.

The antenna changeover selection switch unit 49 is configured using a mechanical switch, a semiconductor switch, or the like. The receiving antenna 40(n) is electrically connected to the antenna changeover selection switch unit 49 via a capacitor C1. If a switching signal Sg1 that switches the receiving antenna 40(n) that receives a wireless signal is input from the control unit 59, the antenna changeover selection switch unit 49 selects the receiving antenna 40(n) instructed by the switching signal Sg1 and outputs a wireless signal received by the selected receiving antenna 40(n) to the transmitting/receiving circuit 50. The capacity of a capacitor connected to the receiving antenna 40(n) is equal to the capacity of the capacitor C1.

The transmitting/receiving circuit 50 performs predetermined processes such as a demodulation process and an amplification process on a wireless signal received by the receiving antenna 40(n) selected by the antenna changeover selection switch unit 49, and outputs the wireless signal to the signal processing circuit 51 and the received field strength detector 52.

The signal processing circuit 51 extracts image data from the wireless signal input from the transmitting/receiving circuit 50, performs predetermined processes such as various image processing and an A/D conversion process on the extracted image data, and outputs the image data to the control unit 59.

The received field strength detector 52 detects the received field strength in accordance with the strength of the wireless signal input from the transmitting/receiving circuit 50, and outputs to the control unit 59 a received signal strength indicator (RSSI) corresponding to the detected received field strength.

The receiving antenna 40(n) is electrically connected to the antenna power changeover selector 53 via a coil L1. The antenna power changeover selector 53 supplies electric power via the antenna cables 43 (43a to 43c) to the receiving antenna 40(n) selected by the antenna changeover selection switch unit 49. The electrical characteristic of the coil connected to the receiving antenna 40(n) is equal to that of the coil L1.

The antenna power changeover selector 53 includes a power changeover selection switch unit 531 and an abnormality detector 532. The power changeover selection switch unit 531 is configured using a mechanical switch, a semiconductor switch, or the like. If a selection signal Sg2 for selecting the receiving antenna 40(n) to which power is supplied is input from the control unit 59, the power changeover selection switch unit 531 selects the receiving antenna 40(n) instructed by the selection signal Sg2, and power is supplied only to the selected receiving antenna 40(n).

If an abnormality is occurring at the receiving antenna 40(n) being a power supply destination, the abnormality detector 532 outputs to the control unit 59 an abnormal signal indicating the occurrence of the abnormality at the receiving antenna 40(n) being the power supply destination.

The display unit 54 is configured using a display panel including liquid crystals, organic EL (Electro Luminescence), or the like. The display unit 54 displays various pieces of information such as an image corresponding to image data captured by the capsule endoscope 3, the operating state of the receiving device 5B, patient information of the subject 2, and an examination date and time.

The operating unit 55 is used when instruction signals to perform such things as changing the image capture cycle of the capsule endoscope 3 are input. If the instruction signal is input via the operating unit 55, the signal processing circuit 51 transmits the instruction signal to the transmitting/receiving circuit 50. The transmitting/receiving circuit 50 modulates the instruction signal and transmits the instruction signal from the receiving antennas 40(1) to 40(3). The signals transmitted from the receiving antennas 40(1) to 40(3) are received by the antenna 39 of the capsule endoscope 3 (see FIG. 2), and demodulated by the transmitting/receiving circuit 37. The circuit board 36 performs an operation such as changing the image capture cycle in response to the instruction signal.

The storage unit 56 is configured using a semiconductor memory such as a flash memory or a RAM (Random Access Memory) provided fixedly in the receiving device 5B. Moreover, the storage unit 56 stores image data captured by the capsule endoscope 3 and various pieces of information associated with the image data, for example, estimated position information of the capsule endoscope 3, received field strength information, and identification information for identifying the receiving antenna that has received a wireless signal. Furthermore, the storage unit 56 stores various programs to be executed by the receiving device 5B, and the like. The storage unit 56 may be provided with a function as a recording medium interface that stores information in an external recording medium such as a memory card and reads the information stored in the recording medium.

The I/F unit 57 has a function as a communication interface, and transmits and receives to and from the information processing device 6 in a mutual direction via the cradle 6a.

The power unit 58 is configured using a battery detachable from the receiving device 5B and a switch unit that switches between an on state and an off state. The power unit 58 supplies driving power necessary for the configuration units of the receiving device 5B in the on state, and stops the driving power supplied to the configuration units of the receiving device 5B in the off state.

The control unit 59 is configured using a CPU (Central Processing Unit), and the like. The control unit 59 reads a program from the storage unit 56 and executes the program, and performs such things as instructing the units included in the receiving device 5B and transferring data to the units to perform centralized control of the operation of the receiving device 5B. The control unit 59 includes a selection controller 591, an abnormal information addition unit 592, a position information estimating unit 593, and a locus calculator 597.

The selection controller 591 executes control to select one receiving antenna 40(n) that receives a wireless signal transmitted from the capsule endoscope 3 and supply power only to the selected receiving antenna 40(n). Specifically, the selection controller 591 selects one receiving antenna 40(n) that is allowed to receive a wireless signal including an image signal transmitted from the capsule endoscope 3 at an image signal receiving timing, at an antenna selecting timing based on the received field strength of the receiving antennas 40(n) detected by the received field strength detector 52. Moreover, the selection controller 591 executes control to supply power only to the selected receiving antenna 40(n) at the image signal receiving timing. In order to sequentially select the receiving antenna 40(n) that is allowed to receive a wireless signal including an image signal from the plurality of receiving antennas 40(n), for example, at intervals of 100 msec as the antennal selecting timing, the selection controller 591 causes the received field strength detector 52 to detect the received field strength of the receiving antennas 40(n), and then drives the antenna changeover selection switch unit 49 to supply power only to the selected one receiving antenna 40(n).

If the abnormality detector 532 detects an abnormality in any of the three receiving antennas 40(n), the abnormal information addition unit 592 adds abnormal information indicating the occurrence of the abnormality in any of the receiving antennas 40(n) to a wireless signal received by the receiving antenna 40(n). Specifically, the abnormal information addition unit 592 adds abnormal information (flag) to image data generated by the signal processing circuit 51 performing signal processing on the wireless signal received by the receiving antenna 40(n), outputs the image data, and stores the image data in the storage unit 56.

The position information estimating unit 593 includes an existing region acquiring unit 594 having a similar function to that of the existing region acquiring unit 621 illustrated in FIG. 3, an orientation estimating unit 595 having a similar function to that of the orientation estimating unit 622, and a position determination unit 596 having a similar function to that of the position determination unit 623.

As described above, according to the second embodiment, the position information estimating unit 593 is provided in the receiving device 5B. Accordingly, it becomes possible to detect the position of the capsule endoscope 3 in the subject 2 with high accuracy and in real time. Moreover, according to the second embodiment, it is possible to cut a calculation amount in the position information estimating unit 593. Accordingly, it becomes possible to detect the position of the capsule endoscope 3 without significantly increasing a load on the receiving device 5B.

Reference Signs List

| | |
|---|---|
| 1, 1A | CAPSULE ENDOSCOPE SYSTEM |
| 2 | SUBJECT |
| 3 | CAPSULE ENDOSCOPE |
| 4, 4A | RECEIVING ANTENNA UNIT |
| 5, 5A, 5B | RECEIVING DEVICE |

| | |
|---|---|
| 6 | INFORMATION PROCESSING DEVICE |
| 6a | CRADLE |
| 6b | OPERATION INPUT DEVICE |
| 30 | CAPSULE-SHAPED CONTAINER |
| 30a | CONTAINER |
| 30b | OPTICAL DOME |
| 32 | OBJECTIVE LENS |
| 33 | LENS FRAME |
| 34 | IMAGING UNIT |
| 35 | LIGHTING UNIT |
| 36 | CIRCUIT BOARD |
| 37 | TRANSMITTING/RECEIVING CIRCUIT |
| 38 | BUTTON CELLS |
| 39 | ANTENNA |
| 40(1) TO 40(8) | RECEIVING ANTENNA |
| 41a to 41c | ANTENNA UNIT |
| 42a to 42c | ACTIVE CIRCUIT |
| 43, 43a to 43c | ANTENNA CABLE |
| 44, 44A | SHEET |
| 49 | ANTENNA CHANGEOVER SELECTION SWITCH UNIT |
| 50 | TRANSMITTING/RECEIVING CIRCUIT |
| 51 | SIGNAL PROCESSING CIRCUIT |
| 52 | RECEIVED FIELD STRENGTH DETECTOR |
| 53 | ANTENNA POWER CHANGEOVER SELECTOR |
| 54 | DISPLAY UNIT |
| 55 | OPERATING UNIT |
| 56, 64 | STORAGE UNIT |
| 57 | I/F UNIT |
| 58 | POWER UNIT |
| 59, 61 | CONTROL UNIT |
| 62, 593 | POSITION INFORMATION ESTIMATING UNIT |
| 63, 597 | LOCUS CALCULATOR |
| 65 | INPUT UNIT |
| 66 | OUTPUT UNIT |
| 66C | DISPLAY UNIT |
| 531 | POWER CHANGEOVER SELECTION SWITCH UNIT |
| 532 | ABNORMALITY DETECTOR |
| 591 | SELECTION CONTROLLER |
| 592 | ABNORMAL INFORMATION ADDITION UNIT |
| 594, 621 | EXISTING REGION ACQUIRING UNIT |
| 595, 622 | ORIENTATION ESTIMATING UNIT |
| 596, 623 | POSITION DETERMINATION UNIT |

## Claims

1. A position detection device (6) for detecting a position of a capsule endoscope (3) in a subject, the capsule endoscope being introduced into the subject and moving in the subject, based on received strength of signals transmitted from the capsule endoscope at a plurality of receiving antennas (40(1), 40(2), 40(3)), the position detection device comprising:

a region acquiring unit (621) configured to obtain distances between the receiving antennas and the capsule endoscope based on the received strength of the signals received by the plurality of receiving antennas, and configured to acquire a region where at least two spheres of a plurality of spheres with the receiving antennas as centers and the distances corresponding respectively to the receiving antennas as radii overlap with one another;

an orientation estimating unit (622) configured to estimate orientations of the capsule endoscope at a plurality of points in a plurality of sub-regions obtained by dividing the region in a lattice form based on positional relationships between the points and the receiving antennas, and the received strength of the signals received by the receiving antennas; and

a position determination unit configured to determine the position of the capsule endoscope in the subject based on the positions of the points and the orientations of the capsule endoscope,

wherein the orientation estimating unit (622) is configured to acquire a candidate region of a point at which a vector representing the orientation of the capsule endoscope passes based on a direction of the receiving antenna (40n), a direction from the point to the receiving antenna, and the received strength at the receiving antenna, and is configured to determine a position where the candidate regions of the point at which the vector passes overlap with one another, the candidate regions being acquired respectively for the plurality of receiving antennas, and

wherein the position determination unit (623) is configured to calculate theoretical values of the received strength of the signals received by the plurality of receiving antennas based on the positions of the points and the orientations of the capsule endoscope in a case of assuming that the capsule endoscope exists at the points, as well as acquiring measured values of the received strength at the plurality of receiving antennas, and is configured to detect a point having a minimum error between the theoretical value and the measured value as the position of the capsule endoscope out of the plurality of points.

2. The position detection device (6) according to claim 1, wherein the error is a sum of absolute values of differences or a sum of squared residuals between the theoretical values and the measured values at the receiving antennas.

3. The position detection device (6) according to claim 1, further comprising a locus calculator (63) configured to calculate a moving locus of the capsule endoscope based on the position of the capsule endoscope determined by the position determination unit.

4. The position detection device (6) according to claim 3, further comprising an image display unit (66c) configured to display the moving locus of the capsule endoscope in the subject, the moving locus being calculated by the locus calculator.

5. A capsule endoscope system comprising:

a capsule endoscope (3) that is introduced into a subject and moves in the subject to acquire image information inside the subject; and

the position detection device (6) according to claim 1.

6. The capsule endoscope system according to claim 5, wherein the receiving antennas are antennas that use only main polarization.

7. The capsule endoscope system according to claim 6, wherein the receiving antennas are dipole antennas.

8. The position detection device (6) according to claim 1, further comprising
an image display unit (66c) configured to acquire the image information and position information indicating the position of the capsule endoscope corresponding to the image information from the receiving device and displays an image corresponding to the image information and the position of the capsule endoscope.

9. A position detecting program executed by a position detection device (6) according to claim 1 for detecting a position of a capsule endoscope (3) in a subject, the capsule endoscope being introduced into the subject and moving in the subject, based on received strength of signals transmitted from the capsule endoscope at a plurality of receiving antennas (40(1), 40(2), 40(3)), the program comprising:

region acquiring step of obtaining distances between receiving antennas and a capsule endoscope that is introduced into a subject and moves in the subject, based on received strength at the receiving antennas upon the receiving antennas receiving signals transmitted from the capsule endoscope, and acquiring a region where at least two spheres of a plurality of spheres with the receiving antennas as centers and the distances corresponding respectively to the receiving antennas as radii overlap with one another;

estimating step of estimating orientations of the capsule endoscope at a plurality of points in a plurality of subregions obtained by dividing the region in a lattice form based on positional relationships between the points and the receiving antennas, and the received strength of the signals received by the receiving antennas; and

position determining step of determining a position of the capsule endoscope in the subject based on the positions of the points and the orientations of the capsule endoscope,
wherein the estimating step of estimating orientations acquires a candidate region of a point at which a vector representing the orientation of the capsule endoscope passes based on a direction of the receiving antenna (40n), a direction from the point to the receiving antenna, and the received strength at the receiving antenna, and determines, as the point at which the vector passes, a position where the candidate regions of the point at which the vector passes, the candidate regions being acquired respectively for the plurality of receiving antennas, overlap with one another, and
wherein the region acquiring step calculates theoretical values of the received strength of the signals received by the plurality of receiving antennas based on the positions of the points and the orientations of the capsule endoscope in a case of assuming that the capsule endoscope exists at the points, as well as acquiring measured values of the received strength at the plurality of receiving antennas, and detects a point having a minimum error between the theoretical value and the measured value as the position of the capsule endoscope out of the plurality of points.

**Patentansprüche**

1. Positionserfassungseinrichtung (6) zur Erfassung einer Position eines Kapselendoskops (3) in einem Subjekt, wobei das Kapselendoskop in das Subjekt eingeführt ist und sich in dem Subjekt bewegt, auf der Basis einer empfangenden Stärke von Signalen, die von dem Kapselendoskop gesendet werden, an einer Mehrzahl von Empfangsantennen (40(1), 40(2), 40(3)), wobei die Positionserfassungseinrichtung umfasst:

   eine Bereichserfassungseinheit (621), die dazu eingerichtet ist, Entfernungen zwischen den Empfangsantennen und dem Kapselendoskop auf der Basis der empfangenen Stärke der von der Mehrzahl von Empfangsantennen empfangenen Signale zu erhalten, und dazu eingerichtet ist, einen Bereich zu erfassen, in dem mindestens zwei Sphären einer Mehrzahl von Sphären mit den Empfangsantennen als Mittelpunkte und den Entfernungen, die jeweils den Empfangsantennen entsprechen, als Radien einander überlappen;
   eine Orientierungsabschätzungseinheit (622), die dazu eingerichtet ist, Orientierungen des Kapselendoskops an einer Mehrzahl von Punkten in einer Mehrzahl von Unterbereichen, die durch Teilen des Bereichs in eine Gitterform erhalten werden, auf der Basis von Positionsbeziehungen zwischen den Punkten und den Empfangsantennen und der empfangenen Stärke der von den Empfangsantennen empfangenen Signale abzuschätzen; und
   eine Positionsbestimmungseinheit, die dazu eingerichtet ist, die Position des Kapselendoskops in dem Subjekt auf der Basis der Positionen der Punkte und der Orientierungen des Kapselendoskops zu bestimmen,
   wobei die Orientierungsabschätzungseinheit (622) dazu eingerichtet ist, einen Kandidatenbereich eines Punkts, an dem ein die Orientierung des Kapselendoskops repräsentierender Vektor vorbeiführt, auf der Basis einer Richtung der Empfangsantenne (40n), einer Richtung von dem Punkt zu der Empfangsantenne und der empfangenen Stärke an der Empfangsantenne zu erfassen, und dazu eingerichtet ist, eine Position zu bestimmen, wo die Kandidatenbereiche des Punkts, an dem der Vektor vorbeiführt, einander überlappen, wobei die Kandidatenbereiche jeweils für die Mehrzahl von Empfangsantennen erfasst werden, und
   wobei die Positionsbestimmungseinheit (623) dazu eingerichtet ist, theoretische Werte der empfangenen Stärke der von der Mehrzahl von Empfangsantennen empfangenen Signale auf der Basis der Positionen der Punkte und der Orientierungen des Kapselendoskops zu berechnen, wenn angenommen wird, dass das Kapselendoskop an den Punkten vorhanden ist, und Messwerte der empfangenen Stärke an der Mehrzahl von Empfangsantennen erfasst werden, und dazu eingerichtet ist, einen Punkt mit einem minimalen Fehler zwischen dem theoretischen Wert und dem Messwert als die Position des Kapselendoskops aus der Mehrzahl von Punkten zu erfassen.

2. Positionserfassungseinrichtung (6) nach Anspruch 1, bei der der Fehler eine Summe von Absolutwerten von Differenzen oder eine Summe von quadrierten Resten zwischen den theoretischen Werten und den Messwerten an den Empfangsantennen ist.

3. Positionserfassungseinrichtung (6) nach Anspruch 1, die ferner eine Positionsberechnungseinrichtung (63) umfasst, die dazu eingerichtet ist, eine sich bewegende Position des Kapselendoskops auf der Basis der von der Positionsbestimmungseinheit bestimmten Position des Kapselendoskops zu berechnen.

4. Positionserfassungseinrichtung (6) nach Anspruch 3, die ferner eine Bildanzeigeeinheit (66c) umfasst, die dazu eingerichtet ist, die sich bewegende Position des Kapselendoskops in dem Subjekt anzuzeigen, wobei die sich bewegende Position durch die Positionsberechnungseinrichtung berechnet wird.

5. Kapselendoskopsystem, das umfasst:

ein Kapselendoskop (3), das in ein Subjekt eingeführt ist und sich in dem Subjekt bewegt, um eine Bildinformation im Inneren des Subjekts aufzunehmen; und
die Positionserfassungseinrichtung (6) nach Anspruch 1.

6. Kapselendoskopsystem nach Anspruch 5, bei dem die Empfangsantennen Antennen sind, die nur eine Hauptpolarisation nutzen.

7. Kapselendoskopsystem nach Anspruch 6, bei dem die Empfangsantennen Dipolantennen sind.

8. Positionserfassungseinrichtung (6) nach Anspruch 1, die ferner umfasst
eine Bildanzeigeeinheit (66c), die dazu eingerichtet ist, die Bildinformation und die Positionsinformation, die die Position des Kapselendoskops entsprechend der Bildinformation von der Empfangseinrichtung anzeigt, zu erfassen und ein der Bildinformation und der Position des Kapselendoskops entsprechendes Bild anzeigt.

9. Positionserfassungsprogramm, das von einer Positionserfassungseinrichtung (6) nach Anspruch 1 ausgeführt wird, zum Erfassen einer Position eines Kapselendoskops (3) in einem Subjekt, wobei das Kapselendoskop in das Subjekt eingeführt ist und sich in dem Subjekt bewegt, auf der Basis einer empfangenden Stärke von Signalen, die von dem Kapselendoskop gesendet werden, an einer Mehrzahl von Empfangsantennen (40(1), 40(2), 40(3)), wobei das Programm umfasst:

einen Bereichserfassungsschritt zum Erhalten von Entfernungen zwischen Empfangsantennen und einem Kapselendoskop, das in ein Subjekt eingeführt ist und sich in dem Subjekt bewegt, auf der Basis einer empfangenen Stärke an den Empfangsantennen, wenn die Empfangsantennen von dem Kapselendoskop gesendete Signale empfangen, und zum Erfassen eines Bereichs, in dem mindestens zwei Sphären einer Mehrzahl von Sphären mit den Empfangsantennen als Mittelpunkte und den Entfernungen, die jeweils den Empfangsantennen entsprechen, als Radien einander überlappen;
einen Abschätzungsschritt zum Abschätzen von Orientierungen des Kapselendoskops an einer Mehrzahl von Punkten in einer Mehrzahl von Unterbereichen, die durch Teilen des Bereichs in eine Gitterform erhalten werden, auf der Basis von Positionsbeziehungen zwischen den Punkten und den Empfangsantennen und der empfangenen Stärke der von den Empfangsantennen empfangenen Signale; und
einen Positionsbestimmungsschritt zum Bestimmen einer Position des Kapselendoskops in dem Subjekt auf der Basis der Positionen der Punkte und der Orientierungen des Kapselendoskops,
wobei der Abschätzungsschritt zum Abschätzen von Orientierungen einen Kandidatenbereich eines Punkts, an dem ein die Orientierung des Kapselendoskops repräsentierender Vektor vorbeiführt, auf der Basis einer Richtung der Empfangsantenne (40n), einer Richtung von dem Punkt zu der Empfangsantenne und der empfangenen Stärke an der Empfangsantenne erfasst, und eine Position, wo die Kandidatenbereiche des Punkts, an dem der Vektor vorbeiführt, einander überlappen, als den Punkt bestimmt, an dem der Vektor vorbeiführt, wobei die Kandidatenbereiche jeweils für die Mehrzahl von Empfangsantennen erfasst werden, und
wobei der Positionsbestimmungsschritt theoretische Werte der empfangenen Stärke der von der Mehrzahl von Empfangsantennen empfangenen Signale auf der Basis der Positionen der Punkte und der Orientierungen des Kapselendoskops berechnet, wenn angenommen wird, dass das Kapselendoskop an den Punkten vorhanden ist, und Messwerte der empfangenen Stärke an der Mehrzahl von Empfangsantennen erfasst und einen Punkt mit einem minimalen Fehler zwischen dem theoretischen Wert und dem Messwert als die Position des Kapselendoskops aus der Mehrzahl von Punkten erfasst.

## Revendications

1. Dispositif (6) de détection de position destiné à détecter une position d'un endoscope (3) de type capsule dans un sujet, l'endoscope de type capsule étant introduit dans le sujet et se déplaçant dans le sujet, en se basant sur une intensité de réception des signaux transmis depuis l'endoscope de type capsule au niveau d'une pluralité d'antennes de réception (40(1), 40(2), 40(3)), le dispositif de détection de position comprenant :

une unité (621) d'acquisition de région configurée pour obtenir des distances entre les antennes de réception et l'endoscope de type capsule en se basant sur l'intensité de réception des signaux reçus par la pluralité d'antennes de réception, et configurée pour acquérir une région dans laquelle au moins deux sphères parmi une pluralité de sphères ayant les antennes de réception pour centres et les distances correspondant respectivement aux antennes de réception en tant que rayons se chevauchent l'une l'autre ;

une unité (622) d'estimation d'orientation configurée pour estimer les orientations de l'endoscope de type capsule au niveau d'une pluralité de points dans une pluralité de sous-régions obtenues en divisant la région sous forme de treillis en se basant sur les relations positionnelles entre les points et les antennes de réception, et l'intensité de réception des signaux reçus par les antennes de réception ; et

une unité de détermination de position configurée pour déterminer la position de l'endoscope de type capsule dans le sujet en se basant sur les positions des points et les orientations de l'endoscope de type capsule,

dans lequel l'unité (622) d'estimation d'orientation est configurée pour acquérir une région candidate d'un point au niveau duquel un vecteur représentant l'orientation de l'endoscope de type capsule passe en se basant sur une direction de l'antenne de réception (40n), une direction allant du point à l'antenne de réception, et l'intensité de réception au niveau de l'antenne de réception, et est configurée pour déterminer une position dans laquelle les régions candidates du point au niveau duquel le vecteur passe se chevauchent l'une l'autre, les régions candidates étant acquises respectivement pour la pluralité d'antennes de réception, et

dans lequel l'unité (623) de détermination de position est configurée pour calculer des valeurs théoriques de l'intensité de réception des signaux reçus par la pluralité d'antennes de réception en se basant sur les positions des points et les orientations de l'endoscope de type capsule dans un cas où on suppose que l'endoscope de type capsule se trouve au niveau des points, de même que l'acquisition de valeurs mesurées de l'intensité de réception au niveau de la pluralité d'antennes de réception, et est configurée pour détecter un point ayant une erreur minimale entre la valeur théorique et la valeur mesurée en tant que position de l'endoscope de type capsule parmi la pluralité de points.

2. Dispositif (6) de détection de position selon la revendication 1, dans lequel l'erreur est une somme des valeurs absolues des différences ou une somme des résidus au carré entre les valeurs théoriques et les valeurs mesurées au niveau des antennes de réception.

3. Dispositif (6) de détection de position selon la revendication 1, comprenant en outre un calculateur (63) d'emplacement configuré pour calculer un emplacement mobile de l'endoscope de type capsule en se basant sur la position de l'endoscope de type capsule déterminée par l'unité de détermination de position.

4. Dispositif (6) de détection de position selon la revendication 3, comprenant en outre une unité (66c) d'affichage d'image configurée pour afficher l'emplacement mobile de l'endoscope de type capsule dans le sujet, l'emplacement mobile étant calculé par le calculateur d'emplacement.

5. Système d'endoscope de type capsule comprenant :

un endoscope (3) de type capsule qui est introduit dans un sujet et se déplace dans le sujet pour acquérir des informations d'image à l'intérieur du sujet ; et

le dispositif (6) de détection de position selon la revendication 1.

6. Système d'endoscope de type capsule selon la revendication 5, dans lequel les antennes de réception sont des antennes qui utilisent uniquement une polarisation principale.

7. Système d'endoscope de type capsule selon la revendication 6, dans lequel les antennes de réception sont des antennes dipôles.

8. Dispositif (6) de détection de position selon la revendication 1, comprenant en outre

une unité (66c) d'affichage d'image configurée pour acquérir les informations d'image et des informations de position indiquant la position de l'endoscope de type capsule correspondant aux informations d'image à partir du dispositif de réception et afficher une image correspondant aux informations d'image et à la position de l'endoscope de type capsule.

9. Programme de détection de position exécuté par un dispositif (6) de détection de position selon la revendication 1, destiné à détecter une position d'un endoscope (3) de type capsule dans un sujet, l'endoscope de type capsule étant introduit dans le sujet et se déplaçant dans le sujet, en se basant sur une intensité de réception des signaux transmis depuis l'endoscope de type capsule au niveau d'une pluralité d'antennes de réception (40(1), 40(2), 40(3)), le programme comprenant :

une étape d'acquisition de région consistant à obtenir les distances entre les antennes de réception et un endoscope de type capsule qui est introduit dans un sujet et se déplace dans le sujet, en se basant sur une intensité de réception au niveau des antennes de réception lorsque les antennes de réception reçoivent les signaux transmis depuis l'endoscope de type capsule, et acquérir une région dans laquelle au moins deux sphères parmi une pluralité de sphères ayant les antennes de réception pour centres et les distances correspondant respectivement aux antennes de réception en tant que rayons se chevauchent l'une l'autre ;

une étape d'estimation consistant à estimer les orientations de l'endoscope de type capsule au niveau d'une pluralité de points dans une pluralité de sous-régions obtenues en divisant la région sous forme de treillis en se basant sur les relations positionnelles entre les points et les antennes de réception, et l'intensité de réception des signaux reçus par les antennes de réception ; et

une étape de détermination de position consistant à déterminer une position de l'endoscope de type capsule dans le sujet en se basant sur les positions des points et les orientations de l'endoscope de type capsule,

dans lequel l'étape d'estimation consistant à estimer les orientations acquiert une région candidate d'un point au niveau duquel un vecteur représentant l'orientation de l'endoscope de type capsule passe en se basant sur une direction de l'antenne de réception (40n), une direction allant du point à l'antenne de réception, et l'intensité de réception au niveau de l'antenne de réception, et détermine, en tant que point au niveau duquel passe le vecteur, une position dans laquelle les régions candidates du point au niveau duquel passe le vecteur, les régions candidates étant acquises respectivement pour la pluralité d'antennes de réception, se chevauchent l'une l'autre, et

dans lequel l'étape d'acquisition de région calcule des valeurs théoriques de l'intensité de réception des signaux reçus par la pluralité d'antennes de réception en se basant sur les positions des points et les orientations de l'endoscope de type capsule dans un cas dans lequel on suppose que l'endoscope de type capsule se trouve au niveau des points, de même que l'acquisition des valeurs mesurées de l'intensité de réception au niveau de la pluralité d'antennes de réception, et détecte un point présentant une erreur minimale entre la valeur théorique et la valeur mesurée en tant que position de l'endoscope de type capsule parmi la pluralité de points.

# FIG.1

CAPSULE ENDOSCOPIC SYSTEM 1

SUBJECT 2

CAPSULE ENDOSCOPE 3

RECEIVING ANTENNA UNIT 4

40(1)

40(2)

40(3)

43

44

RECEIVING DEVICE 5

54

55

5

6a

INFORMATION PROCESSING UNIT 6

66c

6b

# FIG.2

# FIG.3

# FIG.4

START

ACQUIRE PARAMETERS USED FOR ESTIMATION ~S1

ACQUIRE SIGNAL STRENGTH OF RECEIVING ANTENNAS ~S2

POSITION DETERMINATION PROCESS ~S3

MOVING LOCUS CALCULATION PROCESS ~S4

END

EP 2 842 476 B1

# FIG.5

```
┌──────────────────────────────┐
│   POSITION DETERMINATION     │
│         PROCESS              │
└──────────────────────────────┘
              │
              ▼              S11
        ╱───────────────────────╲        NO
       ╱  IS IMAGE TO BE DISPLAYED? ╲──────────┐
        ╲───────────────────────╱              │
              │ YES                             │
              ▼                                 │
┌──────────────────────────────────────┐       │
│ ACQUIRE DISTANCES rₙ BETWEEN RECEIVING│  S12  │
│ ANTENNAS AND CAPSULE ENDOSCOPE        │       │
└──────────────────────────────────────┘       │
              │                                 │
              ▼                                 │
┌──────────────────────────────────────┐       │
│ ACQUIRE EXISTING REGION T OF CAPSULE  │  S13  │
│ ENDOSCOPE                             │       │
└──────────────────────────────────────┘       │
              │                                 │
              ▼                                 │
┌──────────────────────────────────────┐       │
│ LOOP A: PROCESS FOR ALL LATTICE       │       │
│ POINTS Pi IN EXISTING REGION T        │       │
└──────────────────────────────────────┘       │
              │                                 │
              ▼                                 │
┌──────────────────────────────────────┐       │
│ PROCESS OF ESTIMATING ORIENTATION OF  │  S14  │
│ CAPSULE ENDOSCOPE                     │       │
└──────────────────────────────────────┘       │
              │                                 │
              ▼                                 │
┌──────────────────────────────────────┐       │
│ CALCULATE THEORETICAL VALUE Vtₙ(i) OF │       │
│ RECEIVED STRENGTH AT EACH RECEIVING   │  S15  │
│ ANTENNA BASED ON POSITION OF LATTICE  │       │
│ POINT Pi AND ORIENTATION OF CAPSULE   │       │
│ ENDOSCOPE                             │       │
└──────────────────────────────────────┘       │
              │                                 │
              ▼                                 │
┌──────────────────────────────────────┐       │
│ CALCULATE SUM OF ABSOLUTE VALUES OF   │       │
│ DIFFERENCES BETWEEN THEORETICAL VALUES│  S16  │
│ Vtₙ(i) AND MEASURED VALUES Vₙ OF      │       │
│ RECEIVED STRENGTH                     │       │
└──────────────────────────────────────┘       │
              │                                 │
              ▼                                 │
┌──────────────────────────────────────┐       │
│              LOOP A                   │       │
└──────────────────────────────────────┘       │
              │                                 │
              ▼                                 │
┌──────────────────────────────────────┐       │
│ ACQUIRE POSITION AND ORIENTATION THAT │       │
│ HAVE MINIMUM SUM OF ABSOLUTE VALUES OF│  S17  │
│ DIFFERENCES IN EXISTING REGION T      │       │
└──────────────────────────────────────┘       │
              │                                 │
              ▼                                 │
┌──────────────────────────────────────┐       │
│ DETERMINE ACQUIRED POSITION AS        │  S18  │
│ POSITION OF CAPSULE ENDOSCOPE         │       │
└──────────────────────────────────────┘       │
              │                                 │
              ▼◄────────────────────────────────┘
        ┌──────────────────┐
        │      RETURN       │
        └──────────────────┘
```

The flowchart reads:

POSITION DETERMINATION PROCESS

S11: IS IMAGE TO BE DISPLAYED? — NO → RETURN; YES ↓

S12: ACQUIRE DISTANCES $r_n$ BETWEEN RECEIVING ANTENNAS AND CAPSULE ENDOSCOPE

S13: ACQUIRE EXISTING REGION T OF CAPSULE ENDOSCOPE

LOOP A: PROCESS FOR ALL LATTICE POINTS Pi IN EXISTING REGION T

S14: PROCESS OF ESTIMATING ORIENTATION OF CAPSULE ENDOSCOPE

S15: CALCULATE THEORETICAL VALUE $Vt_n(i)$ OF RECEIVED STRENGTH AT EACH RECEIVING ANTENNA BASED ON POSITION OF LATTICE POINT Pi AND ORIENTATION OF CAPSULE ENDOSCOPE

S16: CALCULATE SUM OF ABSOLUTE VALUES OF DIFFERENCES BETWEEN THEORETICAL VALUES $Vt_n(i)$ AND MEASURED VALUES $V_n$ OF RECEIVED STRENGTH

LOOP A

S17: ACQUIRE POSITION AND ORIENTATION THAT HAVE MINIMUM SUM OF ABSOLUTE VALUES OF DIFFERENCES IN EXISTING REGION T

S18: DETERMINE ACQUIRED POSITION AS POSITION OF CAPSULE ENDOSCOPE

RETURN

24

# FIG.6

# FIG.7

# FIG.8

```
┌─────────────────────────────┐
│  PROCESS OF ESTIMATING      │
│  ORIENTATION OF CAPSULE     │
│  ENDOSCOPE                  │
└─────────────────────────────┘
              │
              ▼
┌─────────────────────────────────────┐
│         LOOP B:                      │
│  PROCESS FOR RECEIVING ANTENNAS      │
└─────────────────────────────────────┘
              │
              ▼
┌─────────────────────────────────────┐
│  ACQUIRE VECTOR a FROM ORIENTATION OF│   S21
│  RECEIVING ANTENNA                   │
└─────────────────────────────────────┘
              │
              ▼
┌─────────────────────────────────────┐
│  ACQUIRE VECTOR b FROM POSITIONAL    │
│  RELATIONSHIP BETWEEN CAPSULE        │   S22
│  ENDOSCOPE AND RECEIVING ANTENNA     │
└─────────────────────────────────────┘
              │
              ▼
┌─────────────────────────────────────┐
│  ACQUIRE CROSS PRODUCT c = a × b OF  │   S23
│  VECTORS a AND b                     │
└─────────────────────────────────────┘
              │
              ▼
┌─────────────────────────────────────┐
│  ACQUIRE CIRCUMFERENCE Cn OF CIRCLE  │
│  ORTHOGONAL TO CROSS PRODUCT c FROM  │
│  SPHERICAL SURFACE HAVING CROSS      │   S24
│  PRODUCT c AS RADIUS BASED ON        │
│  RECEIVED STRENGTH OF RECEIVING      │
│  ANTENNA                             │
└─────────────────────────────────────┘
              │
              ▼
┌─────────────────────────────────────┐
│             LOOP B                   │
└─────────────────────────────────────┘
              │
              ▼
┌─────────────────────────────────────┐
│  ACQUIRE ORIENTATION Vc OF CAPSULE   │
│  ENDOSCOPE FROM INTERSECTION POINT   │   S25
│  OF N NUMBER OF ACQUIRED Cn          │
└─────────────────────────────────────┘
              │
              ▼
┌─────────────────────────────┐
│           RETURN            │
└─────────────────────────────┘
```

# FIG.9

# FIG.10

# FIG.11

# FIG.12

EP 2 842 476 B1

# FIG.13

# FIG.14

# FIG.15

# FIG.16

# FIG.17

# FIG.18

CAPSULE ENDOSCOPIC SYSTEM
1A

EP 2 842 476 B1

33

# FIG.19

ANTENNA POWER CHANGEOVER SELECTOR ~53

POWER CHANGEOVER SELECTION SWITCH UNIT ~531

ABNORMALITY DETECTOR ~532

CONTROL UNIT ~59

SELECTION CONTROLLER ~591

ABNORMAL INFORMATION ADDITION UNIT ~592

POSITION INFORMATION ESTIMATING UNIT ~593

EXISTING REGION ACQUIRING UNIT ~594

ORIENTATION ESTIMATING UNIT ~595

POSITION DETERMINATION UNIT ~596

LOCUS CALCULATOR ~597

DISPLAY UNIT ~54

OPERATING UNIT ~55

STORAGE UNIT ~56

I/F UNIT ~57

POWER UNIT ~58

5B

L1

40(1)

41a

42a ACTIVE CIRCUIT ~43a

C1

L1

40(2)

41b

42b ACTIVE CIRCUIT ~43b

C1

L1

40(3)

41c

42c ACTIVE CIRCUIT ~43c

C1

ANTENNA CHANGEOVER SELECTION SWITCH UNIT ~49

Sg2

Sg1

TRANSMITTING/ RECEIVING CIRCUIT ~50

SIGNAL PROCESSING CIRCUIT ~51

RECEIVED FIELD STRENGTH DETECTOR ~52

EP 2 842 476 B1

**EP 2 842 476 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2006183993 A1
- US 2002193685 A1
- US 2008312501 A1

- JP 2008099734 A
- JP 2006068501 A
- JP 2007283001 A